# EUROPEAN PATENT APPLICATION

(11) **EP 1 840 211 A1**
(43) Date of publication of application: **03.10.2007**
(21) Application number: 07012775.8
(22) Date of filing: 30.10.2002
(51) Int. Cl.: C12N 9/88, C12N 15/60

(54) **Pyranosone dehydratase from phanerochaete chrysosporium**

(30) Priority: 31.10.2001 GB 0126164; 21.12.2001 US 343485 P
(62) Divisional of application: 02772583.7
(71) Applicant: DANISCO A/S, 1001 Copenhagen K. (DK)
(72) Inventor: Morgan, Andrew John, Haywards Heath West Sussex RH16 1QP (GB); Yu, Shukun, 212 40 Malmoe (SE); Weiergang, Inge, 1729 Copenhagen V (DK); Pedersen, Hans Christian, 4900 Nakskov (DK)
(74) Representative: Williams, Aylsa

(57) **Abstract**

The present invention discloses sequence information relating to pyranosone dehydratase.

The invention further relates to the use of pyranosone dehydratase in the conversion of AF to APP and microthecin and the conversion of glucosone to cortalcerone.

SEQUENCE LISTING

<110> Danisco A/S

<120> Sequences

<130> P011937EPA

<150> GB 0126164.3
<151> 2001-10-31

<150> US 60/343,485
<151> 2001-12-21

<160> 84

<170> PatentIn version 3.1

<210> 1
<211> 3732
<212> DNA
<213> Phanerochaete chrysosporium

<400> 1
tgtccgatgc cacggagcat ccagtctgga gctatctcgt atgcccttag cgtatctcgt 60

ggtttttctc ggcactcact cctctgcttc tcgcagaccc ttgtcgtcac attttcaaat 120

cagcataatg gaaggcctac atgccaatgc gtaggatatt cattacgtct ctcgcccgag 180

acgagctcct ctcaaggcat tggtcttggt tcaccaatta cagagacgcc gcagaggtgt 240

atatgtgagc agcggagagc tcaccacctt caaacaacca tcgcgacgat gtacagcaaa 300

gtcttcctca agccgcactg tgagcccgag cagcctgccg ctctccctct cttccagccc 360

caactcgtgc agggaggacg tcctgatggc tactgggtcg aggcattccc ctttcgctca 420

gactccagca aatgccccaa catcattggc tatggactcg gcacgtacga catgaagagc 480

gacatccaga tgtttgtcaa cccatacgca actaccaaca atcagtgagt cctcatattt 540

ttttctatga attacggtgg tataatctct cctctagaag ctcgtcttgg acccctgtct 600

cactggcaaa actcgatttc ccggtcgcaa tgcactatgc cgacatcacg aagaatggtt 660

ttaatgatgg tcggtgtatt tttttttttt tttgctatat ctcatgcttt gctaaccatc 720

gcacagttat catcacggac caatacggct cctcgatgga cgacatctgg gcctatggtg 780

gacgcgtcag ctggctcgag aatcccggcg agctgcgcga caattggacg atgcgcacga 840

ttgggcacag cccgggcatg caccggctca aggcggggca cttcacgcgc acggaccgtg 900

tgcaggtcgt cgcagtgccg atcgtcgttg cgtccagcga cctcacgacg ccggcggacg 960

tcatcatctt cactgccccc gacgatcctc gctcagagca gctctggcag cgtgacgtcg 1020

tcggcacgcg ccacctcgtc catgaggtcg ccatcgtccc cgccgccgaa actgatggcg 1080

aaatgcgctt cgaccagatc atccttgcgg gacgcgacgg tgtcgactgc ctgtggtatg 1140

acggcgccag gtggcagaag catctcgtcg gcacgggcct tccggaagag cgcggagacc 1200

cctattgggg tgcgggctcc gctgcggttg gacgcgtagg cgacgactat gcgggataca 1260
tctgctctgc cgaggtaggc tttggctcca tcatttttcg caggtcactt accggtattt 1320
ttgcaggcat tccacggcaa taccgtctcg gtctatacaa agcccgctgg ctcaccgacg 1380
ggcatcgtcc gcgcagagtg gacgagacat gtgctcgacg tcttcgggcc actcaacggg 1440
aagcacaccg ggagcattca ccaggtcgtc tgcgcggaca tcgatggaga cggggaagac 1500
gaatttctcg tagccatgat gggcgcagat cctccggact tccagaggac aggcgtttgg 1560
tgctataagc gtgagttaac ttcggtgtct tcaatgatac agatgctgat tgtgcgctct 1620
ggcagttgtc gacaggacaa acatgaagtt ctccaagacc aaagtcagta gtgtttctgc 1680
cgggcgcatc gcaacagcga acttccactc gcagggctcc gaagtggtgt gtattttgtc 1740
cagcactgac tatgagacag aatattcata cagatctttc taggacattg ccaccatctc 1800
ttactctgtt cctggatatt ttgagtcccc caacccgtcc atcaacgtct tcctctccac 1860
cggcattctt gccgagcggc ttgacgaaga ggtgatgctc agggtggtcc gcgcaggatc 1920
gacgcgcttc aagaccgaga tggagttcct tgacgtcgcg ggaaagaagc ttacgcttgt 1980
cgtgctgccg cccttcgcac gcctcgatgt cgaacgcaat gtgtccggtg tgaaggtcat 2040
ggccgggaca gtctgttggg ccgacgagaa cgggaagcat gaacgcgtgc ctgcaacgcg 2100
cccattcggc tgcgagagca tgatcgtctc cgcagactat ctcgagagcg ggaagagggg 2160
cgcgatcctc gtcctctaca agccctcgag cacctcaggc cggccgccgt tccgttctat 2220
ggacgaactt gtggcgcaca acctgttccc cgcgtacgtc cccgatagtg ttcgcgcgat 2280
gaagttcccc tgggtacgct gcgcagatcg cccgtgggcg catggccgct tcaaggtaat 2340
gtttctcccg cagccccctt gaatagccgt cttcgctgac cctggccatg ataggacctt 2400
gacttcttca acctcatcgg cttccacgtc aactttgcgg atgattccgc ggctgtgctc 2460
gcgcacgttc agctctggac ggcgggcatt ggcgtctccg ctgggttcca caaccacgtc 2520
gaagcgtcgt tctgcgagat ccatgcctgc atcgcgaacg gcaccggtcg cggcgggatg 2580
cgctgggcaa ccgttcccga tgccaatttc aacccagaca gcccgaacct cgaggacacg 2640
gagctgattg tcgtgcctga catgcacgag cacggcccac tctggcgcac gcgtcctgat 2700
ggacacccgc tcctgcgcat gaatgacacc atcgactacc catggcatgg tgcgtgcatg 2760
actaattgcg gcgcacttcc gcgctgacac ggctctgcgt caccagcttg gctggcgggc 2820
gccggcaacc ccagcccgca ggcgttcgac gtctgggttg cgttcgagtt ccccgggttc 2880
gaaacgttct cgactcctcc gcctccgcgc gtactcgagc ccgggaggta cgcaatccgg 2940
tttggagacc ctcaccagac cgcatcgctt gcccttcaga agaacgatgc cacagacggc 3000
acccccgttc tcgcgctcct cgacctcgat ggcggcccgt cgccgcaggc ggtgagtcat 3060
acctcttctg tgctcgcaca tacaagctta catggacact ctcagtggaa tatctctcat 3120

gttcccggca cggacatgta cgagatcgcg cacgccaaga cgggttcgct tgtctgtgct 3180

cgttggccgc ccgttaagaa tcagcgtgtc gccggcacgc actctcctgc tgccatgggt 3240

cttacgtcac ggtgggccgt cacgaagaac accaaggggc agattacgtg cgtaatcccg 3300

ttggtatagc cgcggtcgtg atgctcagtg cttgcatgta gcttccgtct cccggaggcg 3360

cccgaccatg gcccgctctt ccttagcgtt tccgctatac gccaccaaca gggagcagac 3420

gcgattcccg tacgtgatag actgctatcc ctgttcaagt tttgtctcac gtatttacac 3480

tttatcctct caggtcatcg tgcaggggga cagcattgag ctttcggcgt ggtctcttgt 3540

tcctgccaac tgaaaaggta tcttggaaaa ccggttcatg gaatgtttcg ttgtacaata 3600

gtgtatgaag taacaaagct atgtgctacc gccagtggtc ttcgaacgac agcacttgcc 3660

tgaaaaggat gaggggatac gtcacgtgat gaggtgtacg cgcgcgcttg ccgcagactc 3720

aacctgcggc ca 3732

<210> 2
<211> 41
<212> PRT
<213> Phanerochaete chrysosporium

<220>
<221> MISC_FEATURE
<222> (27)..(27)
<223> Xaa is an unknown amino acid residue

<220>
<221> MISC_FEATURE
<222> (27)..(27)
<223> Xaa is Gly in the database

<400> 2
Lys Pro His Cys Glu Pro Glu Gln Pro Ala Ala Leu Pro Leu Phe Gln
1               5                   10                  15

Pro Gln Leu Val Gln Gly Gly Arg Pro Asp Xaa Tyr Trp Val Glu Ala
            20                  25                  30

Phe Pro Phe Arg Ser Asp Ser Ser Lys
        35                  40

<210> 3
<211> 41
<212> PRT
<213> Phanerochaete chrysosporium

<220>
<221> MISC_FEATURE
<222> (19)..(19)
<223> Xaa is an unknown amino acid residue

<220>
<221> MISC_FEATURE
<222> (19)..(19)

<223>  Xaa is Ser in the database sequence

<400>  3
Ser Asp Ile Gln Met Phe Val Asn Pro Tyr Ala Thr Thr Asn Asn Gln
1               5                   10                  15

Ser Ser Xaa Trp Thr Pro Val Ser Leu Ala Lys Leu Asp Phe Pro Val
            20                  25                  30

Ala Met His Tyr Ala Asp Ile Thr Lys
        35                  40

<210>  4
<211>  11
<212>  PRT
<213>  Phanerochaete chrysosporium

<400>  4
Val Ser Trp Leu Glu Asn Pro Gly Glu Leu Arg
1               5                   10

<210>  5
<211>  12
<212>  PRT
<213>  Phanerochaete chrysosporium

<400>  5
Asp Gly Val Asp Cys Leu Trp Tyr Asp Gly Ala Arg
1               5                   10

<210>  6
<211>  27
<212>  PRT
<213>  Phanerochaete chrysosporium

<220>
<221>  MISC_FEATURE
<222>  (23)..(23)
<223>  Xaa is an unknown amino acid residue

<220>
<221>  MISC_FEATURE
<222>  (23)..(23)
<223>  Xaa is Pro in the database

<220>
<221>  MISC_FEATURE
<222>  (25)..(26)
<223>  Xaa is an unknown amino acid residue

<220>
<221>  MISC_FEATURE
<222>  (25)..(25)
<223>  Xaa is Asn in the database

<220>
<221>  MISC_FEATURE
<222>  (26)..(26)
<223>  Xaa is Gly in the database

<400>  6
Pro Ala Gly Ser Pro Thr Gly Ile Val Arg Ala Glu Trp Thr Arg His
1               5                   10                  15

Val Leu Asp Val Phe Gly Xaa Leu Xaa Xaa Lys
            20                  25

<210>  7
<211>  42
<212>  PRT
<213>  Phanerochaete chrysosporium

<400>  7
His Thr Gly Ser Ile His Gln Val Val Cys Ala Asp Ile Asp Gly Asp
1               5                   10                  15

Gly Glu Asp Glu Phe Leu Val Ala Met Met Gly Ala Asp Pro Pro Asp
            20                  25                  30

Phe Gln Arg Thr Gly Val Trp Cys Tyr Lys
        35                  40

<210>  8
<211>  11
<212>  PRT
<213>  Phanerochaete chrysosporium

<400>  8
Thr Glu Met Glu Phe Leu Asp Val Ala Gly Lys
1               5                   10

<210>  9
<211>  23
<212>  PRT
<213>  Phanerochaete chrysosporium

<400>  9
Lys Leu Thr Leu Val Val Leu Pro Pro Phe Ala Arg Leu Asp Val Glu
1               5                   10                  15

Arg Asn Val Ser Gly Val Lys
            20

<210>  10
<211>  20
<212>  PRT
<213>  Phanerochaete chrysosporium

<400>  10
Ser Met Asp Glu Leu Val Ala His Asn Leu Phe Pro Ala Tyr Val Pro
1               5                   10                  15

Asp Ser Val Arg
            20

<210>  11
<211>  42
<212>  PRT
<213>  Phanerochaete chrysosporium

<400>  11
Asn Asp Ala Thr Asp Gly Thr Pro Val Leu Ala Leu Leu Asp Leu Asp
1               5                   10                  15

Gly Gly Pro Ser Pro Gln Ala Trp Asn Ile Ser His Val Pro Pro Gly
            20                  25                  30

Thr Asp Met Tyr Glu Ile Ala His Ala Lys
        35                  40

<210>  12
<211>  13
<212>  PRT
<213>  Phanerochaete chrysosporium

<400>  12
Thr Gly Ser Leu Val Cys Ala Arg Trp Pro Pro Val Lys
1               5                   10

<210>  13
<211>  23
<212>  PRT
<213>  Phanerochaete chrysosporium

<400>  13
Asn Gln Arg Val Ala Gly Thr His Ser Pro Ala Ala Met Gly Leu Thr
1               5                   10                  15

Ser Arg Trp Ala Val Thr Lys
            20

<210>  14
<211>  26
<212>  PRT
<213>  Phanerochaete chrysosporium

<400>  14
Gly Gln Ile Thr Phe Arg Leu Pro Glu Ala Pro Asp His Gly Pro Leu
1               5                   10                  15

Phe Leu Ser Val Ser Ala Ile Arg His Gln
            20                  25

<210>  15
<211>  28
<212>  PRT
<213>  Phanerochaete chrysosporium

<220>

<221>  MISC_FEATURE
<222>  (4)..(4)
<223>  Xaa is an unknown amino acid residue

<220>
<221>  MISC_FEATURE
<222>  (27)..(27)
<223>  Xaa is an unknown amino acid residue

<400>  15
Lys Pro His Xaa Glu Pro Glu Gln Pro Ala Ala Leu Pro Leu Phe Gln
1               5                   10                  15

Pro Gln Leu Val Val Gly Gly Arg Pro Asp Xaa Tyr
            20                  25

<210>  16
<211>  28
<212>  PRT
<213>  Phanerochaete chrysosporium

<220>
<221>  MISC_FEATURE
<222>  (4)..(4)
<223>  Xaa is an unknown amino acid residue

<220>
<221>  MISC_FEATURE
<222>  (27)..(27)
<223>  Xaa is an unknown amino acid residue

<400>  16
Lys Pro His Xaa Glu Pro Glu Gln Pro Ala Ala Leu Pro Leu Phe Gln
1               5                   10                  15

Pro Gln Leu Val Gln Gly Gly Arg Pro Asp Xaa Tyr
            20                  25

<210>  17
<211>  29
<212>  PRT
<213>  Phanerochaete chrysosporium

<220>
<221>  MISC_FEATURE
<222>  (4)..(4)
<223>  Xaa is an unknown amino acid residue

<220>
<221>  MISC_FEATURE
<222>  (28)..(28)
<223>  Xaa is an unknown amino acid residue

<400>  17
Lys Pro His Xaa Glu Pro Glu Gln Pro Ala Ala Leu Pro Leu Phe Gln
1               5                   10                  15

Pro Gln Leu Val Val Gln Gly Gly Arg Pro Asp Xaa Tyr
            20                  25

<210>  18
<211>  41
<212>  PRT
<213>  Phanerochaete chrysosporium

<220>
<221>  MISC_FEATURE
<222>  (27)..(27)
<223>  Xaa is an unknown amino acid residue

<220>
<221>  MISC_FEATURE
<222>  (27)..(27)
<223>  Xaa is Gly in the database

<400>  18
Lys Pro His Cys Glu Pro Glu Gln Pro Ala Ala Leu Pro Leu Phe Gln
1               5                   10                  15

Pro Gln Leu Val Val Gly Gly Arg Pro Asp Xaa Tyr Trp Val Glu Ala
            20                  25                  30

Phe Pro Phe Arg Ser Asp Ser Ser Lys
        35                  40

<210>  19
<211>  41
<212>  PRT
<213>  Phanerochaete chrysosporium

<220>
<221>  MISC_FEATURE
<222>  (19)..(19)
<223>  Xaa is an unknown amino acid residue

<220>
<221>  MISC_FEATURE
<222>  (19)..(19)
<223>  Xaa is Ser in the database sequence

<400>  19
Ser Asp Ile Gln Asp Phe Val Asn Pro Tyr Ala Thr Thr Asn Asn Gln
1               5                   10                  15

Ser Ser Xaa Trp Thr Pro Val Ser Leu Ala Lys Leu Asp Phe Pro Val
            20                  25                  30

Ala Met His Tyr Ala Asp Ile Thr Lys
        35                  40

<210>  20
<211>  175
<212>  PRT
<213>  Phanerochaete chrysosporium

<400>  20

Cys Pro Met Pro Arg Ser Ile Gln Ser Gly Ala Ile Ser Tyr Ala Leu
1               5                   10                  15

Ser Val Ser Arg Gly Phe Ser Arg His Ser Leu Leu Cys Phe Ser Gln
            20                  25                  30

Thr Leu Val Val Thr Phe Ser Asn Gln His Asn Gly Arg Pro Thr Cys
        35                  40                  45

Gln Cys Val Gly Tyr Ser Leu Arg Leu Ser Pro Glu Thr Ser Ser Ser
    50                  55                  60

Gln Gly Ile Gly Leu Gly Ser Pro Ile Thr Glu Thr Pro Gln Arg Cys
65                  70                  75                  80

Ile Cys Glu Gln Arg Arg Ala His His Leu Gln Thr Thr Ile Ala Thr
                85                  90                  95

Met Tyr Ser Lys Val Phe Leu Lys Pro His Cys Glu Pro Glu Gln Pro
            100                 105                 110

Ala Ala Leu Pro Leu Phe Gln Pro Gln Leu Val Gln Gly Gly Arg Pro
        115                 120                 125

Asp Gly Tyr Trp Val Glu Ala Phe Pro Phe Arg Ser Asp Ser Ser Lys
    130                 135                 140

Cys Pro Asn Ile Ile Gly Tyr Gly Leu Gly Thr Tyr Asp Met Lys Ser
145                 150                 155                 160

Asp Ile Gln Met Phe Val Asn Pro Tyr Ala Thr Thr Asn Asn Gln
                165                 170                 175

<210>  21
<211>  236
<212>  PRT
<213>  Phanerochaete chrysosporium

<400>  21
Val Leu Ile Phe Phe Ser Met Asn Tyr Gly Gly Ile Ile Ser Pro Leu
1               5                   10                  15

Glu Ala Arg Leu Gly Pro Leu Ser His Trp Gln Asn Ser Ile Ser Arg
            20                  25                  30

Ser Gln Cys Thr Met Pro Thr Ser Arg Arg Met Val Leu Met Met Val
        35                  40                  45

Gly Val Phe Phe Phe Phe Phe Ala Ile Ser His Ala Leu Leu Thr Ile
    50                  55                  60

Ala Gln Leu Ser Ser Arg Thr Asn Thr Ala Pro Arg Trp Thr Thr Ser
65                  70                  75                  80

Gly Pro Met Val Asp Ala Ser Ala Gly Ser Arg Ile Pro Ala Ser Cys
                85                  90                  95

Ala Thr Ile Gly Arg Cys Ala Arg Leu Gly Thr Ala Arg Ala Cys Thr
            100                 105                 110

Gly Ser Arg Arg Gly Thr Ser Arg Ala Arg Thr Val Cys Arg Ser Ser
        115                 120                 125

Gln Cys Arg Ser Ser Leu Arg Pro Ala Thr Ser Arg Arg Arg Arg Thr
    130                 135                 140

Ser Ser Ser Ser Leu Pro Pro Thr Ile Leu Ala Gln Ser Ser Ser Gly
145                 150                 155                 160

Ser Val Thr Ser Ser Ala Arg Ala Thr Ser Ser Met Arg Ser Pro Ser
                165                 170                 175

Ser Pro Pro Pro Lys Leu Met Ala Lys Cys Ala Ser Thr Arg Ser Ser
            180                 185                 190

Leu Arg Asp Ala Thr Val Ser Thr Ala Cys Gly Met Thr Ala Pro Gly
        195                 200                 205

Gly Arg Ser Ile Ser Ser Ala Arg Ala Phe Arg Lys Ser Ala Glu Thr
    210                 215                 220

Pro Ile Gly Val Arg Ala Pro Leu Arg Leu Asp Ala
225                 230                 235

<210>  22
<211>  12
<212>  PRT
<213>  Phanerochaete chrysosporium

<400>  22
Ala Thr Thr Met Arg Asp Thr Ser Ala Leu Pro Arg
1               5                   10

<210>  23
<211>  130
<212>  PRT
<213>  Phanerochaete chrysosporium

<400>  23
Ala Leu Ala Pro Ser Phe Phe Ala Gly His Leu Pro Val Phe Leu Gln
1               5                   10                  15

Ala Phe His Gly Asn Thr Val Ser Val Tyr Thr Lys Pro Ala Gly Ser
            20                  25                  30

Pro Thr Gly Ile Val Arg Ala Glu Trp Thr Arg His Val Leu Asp Val
        35                  40                  45

Phe Gly Pro Leu Asn Gly Lys His Thr Gly Ser Ile His Gln Val Val
    50                  55                  60

Cys Ala Asp Ile Asp Gly Asp Gly Glu Asp Glu Phe Leu Val Ala Met
65                  70                  75                  80

Met Gly Ala Asp Pro Pro Asp Phe Gln Arg Thr Gly Val Trp Cys Tyr
                85                  90                  95

Lys Arg Glu Leu Thr Ser Val Ser Ser Met Ile Gln Met Leu Ile Val
            100                 105                 110

Arg Ser Gly Ser Cys Arg Gln Asp Lys His Glu Val Leu Gln Asp Gln
        115                 120                 125

Ser Gln
    130

<210>  24
<211>  25
<212>  PRT
<213>  Phanerochaete chrysosporium

<400>  24
Cys Phe Cys Arg Ala His Arg Asn Ser Glu Leu Pro Leu Ala Gly Leu
1               5                   10                  15

Arg Ser Gly Val Tyr Phe Val Gln His
            20                  25

<210>  25
<211>  22
<212>  PRT
<213>  Phanerochaete chrysosporium

<400>  25
Asp Arg Ile Phe Ile Gln Ile Phe Leu Gly His Cys His His Leu Leu
1               5                   10                  15

Leu Cys Ser Trp Ile Phe
            20

<210>  26
<211>  19

<212>  PRT
<213>  Phanerochaete chrysosporium

<400>  26
Val Pro Gln Pro Val His Gln Arg Leu Pro Leu His Arg His Ser Cys
1               5                   10                  15

Arg Ala Ala

<210>  27
<211>  22
<212>  PRT
<213>  Phanerochaete chrysosporium

<400>  27
Arg Arg Gly Asp Ala Gln Gly Gly Pro Arg Arg Ile Asp Ala Leu Gln
1               5                   10                  15

Asp Arg Asp Gly Val Pro
            20

<210>  28
<211>  42
<212>  PRT
<213>  Phanerochaete chrysosporium

<400>  28
Arg Arg Gly Lys Glu Ala Tyr Ala Cys Arg Ala Ala Ala Leu Arg Thr
1               5                   10                  15

Pro Arg Cys Arg Thr Gln Cys Val Arg Cys Glu Gly His Gly Arg Asp
            20                  25                  30

Ser Leu Leu Gly Arg Arg Glu Arg Glu Ala
        35                  40

<210>  29
<211>  61
<212>  PRT
<213>  Phanerochaete chrysosporium

<400>  29
Thr Arg Ala Cys Asn Ala Pro Ile Arg Leu Arg Glu His Asp Arg Leu
1               5                   10                  15

Arg Arg Leu Ser Arg Glu Arg Gly Arg Gly Arg Asp Pro Arg Pro Leu
            20                  25                  30

Gln Ala Leu Glu His Leu Arg Pro Ala Ala Val Pro Phe Tyr Gly Arg
        35                  40                  45

Thr Cys Gly Ala Gln Pro Val Pro Arg Val Arg Pro Arg
    50                  55                  60

<210> 30
<211> 36
<212> PRT
<213> Phanerochaete chrysosporium

<400> 30
Cys Ser Arg Asp Glu Val Pro Leu Gly Thr Leu Arg Arg Ser Pro Val
1               5                   10                  15

Gly Ala Trp Pro Leu Gln Gly Asn Val Ser Pro Ala Ala Pro Leu Asn
            20                  25                  30

Ser Arg Leu Arg
        35

<210> 31
<211> 130
<212> PRT
<213> Phanerochaete chrysosporium

<400> 31
Asp Leu Asp Phe Phe Asn Leu Ile Gly Phe His Val Asn Phe Ala Asp
1               5                   10                  15

Asp Ser Ala Ala Val Leu Ala His Val Gln Leu Trp Thr Ala Gly Ile
            20                  25                  30

Gly Val Ser Ala Gly Phe His Asn His Val Glu Ala Ser Phe Cys Glu
        35                  40                  45

Ile His Ala Cys Ile Ala Asn Gly Thr Gly Arg Gly Gly Met Arg Trp
    50                  55                  60

Ala Thr Val Pro Asp Ala Asn Phe Asn Pro Asp Ser Pro Asn Leu Glu
65                  70                  75                  80

Asp Thr Glu Leu Ile Val Val Pro Asp Met His Glu His Gly Pro Leu
                85                  90                  95

Trp Arg Thr Arg Pro Asp Gly His Pro Leu Leu Arg Met Asn Asp Thr
            100                 105                 110

Ile Asp Tyr Pro Trp His Gly Ala Cys Met Thr Asn Cys Gly Ala Leu
        115                 120                 125

Pro Arg
    130

<210> 32
<211> 173
<212> PRT

<213>  Phanerochaete chrysosporium

<400>  32
His Gly Ser Ala Ser Pro Ala Trp Leu Ala Gly Ala Gly Asn Pro Ser
1               5                   10                  15

Pro Gln Ala Phe Asp Val Trp Val Ala Phe Glu Phe Pro Gly Phe Glu
            20                  25                  30

Thr Phe Ser Thr Pro Pro Pro Pro Arg Val Leu Glu Pro Gly Arg Tyr
        35                  40                  45

Ala Ile Arg Phe Gly Asp Pro His Gln Thr Ala Ser Leu Ala Leu Gln
    50                  55                  60

Lys Asn Asp Ala Thr Asp Gly Thr Pro Val Leu Ala Leu Leu Asp Leu
65                  70                  75                  80

Asp Gly Gly Pro Ser Pro Gln Ala Val Ser His Thr Ser Ser Val Leu
                85                  90                  95

Ala His Thr Ser Leu His Gly His Ser Gln Trp Asn Ile Ser His Val
            100                 105                 110

Pro Gly Thr Asp Met Tyr Glu Ile Ala His Ala Lys Thr Gly Ser Leu
        115                 120                 125

Val Cys Ala Arg Trp Pro Pro Val Lys Asn Gln Arg Val Ala Gly Thr
    130                 135                 140

His Ser Pro Ala Ala Met Gly Leu Thr Ser Arg Trp Ala Val Thr Lys
145                 150                 155                 160

Asn Thr Lys Gly Gln Ile Thr Cys Val Ile Pro Leu Val
                165                 170

<210>  33
<211>  65
<212>  PRT
<213>  Phanerochaete chrysosporium

<400>  33
Cys Ser Val Leu Ala Cys Ser Phe Arg Leu Pro Glu Ala Pro Asp His
1               5                   10                  15

Gly Pro Leu Phe Leu Ser Val Ser Ala Ile Arg His Gln Gln Gly Ala
            20                  25                  30

Asp Ala Ile Pro Val Arg Asp Arg Leu Leu Ser Leu Phe Lys Phe Cys
        35                  40                  45

Leu Thr Tyr Leu His Phe Ile Leu Ser Gly His Arg Ala Gly Gly Gln
    50                  55                  60

His
65

<210> 34
<211> 47
<212> PRT
<213> Phanerochaete chrysosporium

<400> 34
Ala Phe Gly Val Val Ser Cys Ser Cys Gln Leu Lys Arg Tyr Leu Gly
1               5                   10                  15

Lys Pro Val His Gly Met Phe Arg Cys Thr Ile Val Tyr Glu Val Thr
            20                  25                  30

Lys Leu Cys Ala Thr Ala Ser Gly Leu Arg Thr Thr Ala Leu Ala
        35                  40                  45

<210> 35
<211> 20
<212> PRT
<213> Phanerochaete chrysosporium

<400> 35
Gly Asp Thr Ser Arg Asp Glu Val Tyr Ala Arg Ala Cys Arg Arg Leu
1               5                   10                  15

Asn Leu Arg Pro
            20

<210> 36
<211> 50
<212> PRT
<213> Phanerochaete chrysosporium

<400> 36
Val Arg Cys His Gly Ala Ser Ser Leu Glu Leu Ser Arg Met Pro Leu
1               5                   10                  15

Ala Tyr Leu Val Val Phe Leu Gly Thr His Ser Ser Ala Ser Arg Arg
            20                  25                  30

Pro Leu Ser Ser His Phe Gln Ile Ser Ile Met Glu Gly Leu His Ala
        35                  40                  45

Asn Ala
    50

<210>  37
<211>  106
<212>  PRT
<213>  Phanerochaete chrysosporium

<400>  37
Asp Ile His Tyr Val Ser Arg Pro Arg Arg Ala Pro Leu Lys Ala Leu
1               5                   10                  15

Val Leu Val His Gln Leu Gln Arg Arg Arg Arg Gly Val Tyr Val Ser
            20                  25                  30

Ser Gly Glu Leu Thr Thr Phe Lys Gln Pro Ser Arg Arg Cys Thr Ala
        35                  40                  45

Lys Ser Ser Ser Ser Arg Thr Val Ser Pro Ser Ser Leu Pro Leu Ser
    50                  55                  60

Leu Ser Ser Ser Pro Asn Ser Cys Arg Glu Asp Val Leu Met Ala Thr
65                  70                  75                  80

Gly Ser Arg His Ser Pro Phe Ala Gln Thr Pro Ala Asn Ala Pro Thr
                85                  90                  95

Ser Leu Ala Met Asp Ser Ala Arg Thr Thr
            100                 105

<210>  38
<211>  24
<212>  PRT
<213>  Phanerochaete chrysosporium

<400>  38
Arg Ala Thr Ser Arg Cys Leu Ser Thr His Thr Gln Leu Pro Thr Ile
1               5                   10                  15

Ser Glu Ser Ser Tyr Phe Phe Leu
            20

<210>  39
<211>  4
<212>  PRT
<213>  Phanerochaete chrysosporium

<400>  39
Ile Thr Val Val
1

<210>  40
<211>  28
<212>  PRT
<213>  Phanerochaete chrysosporium

<400>  40

Lys Leu Val Leu Asp Pro Cys Leu Thr Gly Lys Thr Arg Phe Pro Gly
1               5                   10                  15

Arg Asn Ala Leu Cys Arg His His Glu Glu Trp Phe
            20                  25

<210>  41
<211>  15
<212>  PRT
<213>  Phanerochaete chrysosporium

<400>  41
Trp Ser Val Tyr Phe Phe Phe Phe Leu Leu Tyr Leu Met Leu Cys
1               5                   10                  15

<210>  42
<211>  99
<212>  PRT
<213>  Phanerochaete chrysosporium

<400>  42
Pro Ser His Ser Tyr His His Gly Pro Ile Arg Leu Leu Asp Gly Arg
1               5                   10                  15

His Leu Gly Leu Trp Trp Thr Arg Gln Leu Ala Arg Glu Ser Arg Arg
            20                  25                  30

Ala Ala Arg Gln Leu Asp Asp Ala His Asp Trp Ala Gln Pro Gly His
        35                  40                  45

Ala Pro Ala Gln Gly Gly Ala Leu His Ala His Gly Pro Cys Ala Gly
    50                  55                  60

Arg Arg Ser Ala Asp Arg Arg Cys Val Gln Arg Pro His Asp Ala Gly
65                  70                  75                  80

Gly Arg His His Leu His Cys Pro Arg Arg Ser Ser Leu Arg Ala Ala
                85                  90                  95

Leu Ala Ala

<210>  43
<211>  9
<212>  PRT
<213>  Phanerochaete chrysosporium

<400>  43
Arg Arg Arg His Ala Pro Pro Arg Pro
1               5

<210>  44
<211>  9

<212>  PRT
<213>  Phanerochaete chrysosporium

<400>  44
Gly Arg His Arg Pro Arg Arg Arg Asn
1               5


<210>  45
<211>  21
<212>  PRT
<213>  Phanerochaete chrysosporium

<400>  45
Trp Arg Asn Ala Leu Arg Pro Asp His Pro Cys Gly Thr Arg Arg Cys
1               5                   10                  15


Arg Leu Pro Val Val
            20


<210>  46
<211>  123
<212>  PRT
<213>  Phanerochaete chrysosporium

<400>  46
Arg Arg Gln Val Ala Glu Ala Ser Arg Arg His Gly Pro Ser Gly Arg
1               5                   10                  15


Ala Arg Arg Pro Leu Leu Gly Cys Gly Leu Arg Cys Gly Trp Thr Arg
            20                  25                  30


Arg Arg Arg Leu Cys Gly Ile His Leu Leu Cys Arg Gly Arg Leu Trp
        35                  40                  45


Leu His His Phe Ser Gln Val Thr Tyr Arg Tyr Phe Cys Arg His Ser
    50                  55                  60


Thr Ala Ile Pro Ser Arg Ser Ile Gln Ser Pro Leu Ala His Arg Arg
65                  70                  75                  80


Ala Ser Ser Ala Gln Ser Gly Arg Asp Met Cys Ser Thr Ser Ser Gly
                85                  90                  95


His Ser Thr Gly Ser Thr Pro Gly Ala Phe Thr Arg Ser Ser Ala Arg
            100                 105                 110


Thr Ser Met Glu Thr Gly Lys Thr Asn Phe Ser
        115                 120


<210>  47
<211>  19
<212>  PRT
<213>  Phanerochaete chrysosporium

<400>  47
Trp Ala Gln Ile Leu Arg Thr Ser Arg Gly Gln Ala Phe Gly Ala Ile
1               5                   10                  15

Ser Val Ser

<210>  48
<211>  5
<212>  PRT
<213>  Phanerochaete chrysosporium

<400>  48
Leu Arg Cys Leu Gln
1               5

<210>  49
<211>  57
<212>  PRT
<213>  Phanerochaete chrysosporium

<400>  49
Leu Cys Ala Leu Ala Val Val Asp Arg Thr Asn Met Lys Phe Ser Lys
1               5                   10                  15

Thr Lys Val Ser Ser Val Ser Ala Gly Arg Ile Ala Thr Ala Asn Phe
            20                  25                  30

His Ser Gln Gly Ser Glu Val Val Cys Ile Leu Ser Ser Thr Asp Tyr
        35                  40                  45

Glu Thr Glu Tyr Ser Tyr Arg Ser Phe
    50                  55

<210>  50
<211>  192
<212>  PRT
<213>  Phanerochaete chrysosporium

<400>  50
Asp Ile Ala Thr Ile Ser Tyr Ser Val Pro Gly Tyr Phe Glu Ser Pro
1               5                   10                  15

Asn Pro Ser Ile Asn Val Phe Leu Ser Thr Gly Ile Leu Ala Glu Arg
            20                  25                  30

Leu Asp Glu Glu Val Met Leu Arg Val Val Arg Ala Gly Ser Thr Arg
        35                  40                  45

Phe Lys Thr Glu Met Glu Phe Leu Asp Val Ala Gly Lys Lys Leu Thr
    50                  55                  60

Leu Val Val Leu Pro Pro Phe Ala Arg Leu Asp Val Glu Arg Asn Val
65                  70                  75                  80

Ser Gly Val Lys Val Met Ala Gly Thr Val Cys Trp Ala Asp Glu Asn
                85                  90                  95

Gly Lys His Glu Arg Val Pro Ala Thr Arg Pro Phe Gly Cys Glu Ser
            100                 105                 110

Met Ile Val Ser Ala Asp Tyr Leu Glu Ser Gly Glu Glu Gly Ala Ile
        115                 120                 125

Leu Val Leu Tyr Lys Pro Ser Ser Thr Ser Gly Arg Pro Pro Phe Arg
    130                 135                 140

Ser Met Asp Glu Leu Val Ala His Asn Leu Phe Pro Ala Tyr Val Pro
145                 150                 155                 160

Asp Ser Val Arg Ala Met Lys Phe Pro Trp Val Arg Cys Ala Asp Arg
                165                 170                 175

Pro Trp Ala His Gly Arg Phe Lys Val Met Phe Leu Pro Gln Pro Pro
            180                 185                 190

<210>  51
<211>  94
<212>  PRT
<213>  Phanerochaete chrysosporium

<400>  51
Ile Ala Val Phe Ala Asp Pro Gly His Asp Arg Thr Leu Thr Ser Ser
1               5                   10                  15

Thr Ser Ser Ala Ser Thr Ser Thr Leu Arg Met Ile Pro Arg Leu Cys
            20                  25                  30

Ser Arg Thr Phe Ser Ser Gly Arg Arg Ala Leu Ala Ser Pro Leu Gly
        35                  40                  45

Ser Thr Thr Thr Ser Lys Arg Arg Ser Ala Arg Ser Met Pro Ala Ser
    50                  55                  60

Arg Thr Ala Pro Val Ala Ala Gly Cys Ala Gly Gln Pro Phe Pro Met
65                  70                  75                  80

Pro Ile Ser Thr Gln Thr Ala Arg Thr Ser Arg Thr Arg Ser
                85                  90

<210>  52
<211>  24

<212>  PRT
<213>  Phanerochaete chrysosporium

<400>  52
Leu Ser Cys Leu Thr Cys Thr Ser Thr Ala His Ser Gly Ala Arg Val
1               5                   10                  15

Leu Met Asp Thr Arg Ser Cys Ala
            20

<210>  53
<211>  12
<212>  PRT
<213>  Phanerochaete chrysosporium

<400>  53
Met Thr Pro Ser Thr Thr His Gly Met Val Arg Ala
1               5                   10

<210>  54
<211>  97
<212>  PRT
<213>  Phanerochaete chrysosporium

<400>  54
Leu Ile Ala Ala His Phe Arg Ala Asp Thr Ala Leu Arg His Gln Leu
1               5                   10                  15

Gly Trp Arg Ala Pro Ala Thr Pro Ala Arg Arg Arg Ser Thr Ser Gly
            20                  25                  30

Leu Arg Ser Ser Ser Pro Gly Ser Lys Arg Ser Arg Leu Leu Arg Leu
        35                  40                  45

Arg Ala Tyr Ser Ser Pro Gly Gly Thr Gln Ser Gly Leu Glu Thr Leu
    50                  55                  60

Thr Arg Pro His Arg Leu Pro Phe Arg Arg Thr Met Pro Gln Thr Ala
65                  70                  75                  80

Pro Pro Phe Ser Arg Ser Ser Thr Ser Met Ala Ala Arg Arg Arg Arg
                85                  90                  95

Arg

<210>  55
<211>  79
<212>  PRT
<213>  Phanerochaete chrysosporium

<400>  55
Val Ile Pro Leu Leu Cys Ser His Ile Gln Ala Tyr Met Asp Thr Leu
1               5                   10                  15

Ser Gly Ile Ser Leu Met Phe Pro Ala Arg Thr Cys Thr Arg Ser Arg
            20                  25                  30

Thr Pro Arg Arg Val Arg Leu Ser Val Leu Val Gly Arg Pro Leu Arg
        35                  40                  45

Ile Ser Val Ser Pro Ala Arg Thr Leu Leu Leu Pro Trp Val Leu Arg
    50                  55                  60

His Gly Gly Pro Ser Arg Arg Thr Pro Arg Gly Arg Leu Arg Ala
65                  70                  75

<210>  56
<211>  85
<212>  PRT
<213>  Phanerochaete chrysosporium

<400>  56
Ser Arg Trp Tyr Ser Arg Gly Arg Asp Ala Gln Cys Leu His Val Ala
1               5                   10                  15

Ser Val Ser Arg Arg Arg Pro Thr Met Ala Arg Ser Ser Leu Ala Phe
            20                  25                  30

Pro Leu Tyr Ala Thr Asn Arg Glu Gln Thr Arg Phe Pro Tyr Val Ile
        35                  40                  45

Asp Cys Tyr Pro Cys Ser Ser Phe Val Ser Arg Ile Tyr Thr Leu Ser
    50                  55                  60

Ser Gln Val Ile Val Gln Gly Asp Ser Ile Glu Leu Ser Ala Trp Ser
65                  70                  75                  80

Leu Val Pro Ala Asn
                85

<210>  57
<211>  15
<212>  PRT
<213>  Phanerochaete chrysosporium

<400>  57
Lys Gly Ile Leu Glu Asn Arg Phe Met Glu Cys Phe Val Val Gln
1               5                   10                  15

<210>  58
<211>  39
<212>  PRT
<213>  Phanerochaete chrysosporium

<400>  58

Gln Ser Tyr Val Leu Pro Pro Val Val Phe Glu Arg Gln His Leu Pro
1               5                   10                  15

Glu Lys Asp Glu Gly Ile Arg His Val Met Arg Cys Thr Arg Ala Leu
            20                  25                  30

Ala Ala Asp Ser Thr Cys Gly
        35

<210>  59
<211>  15
<212>  PRT
<213>  Phanerochaete chrysosporium

<400>  59
Ser Asp Ala Thr Glu His Pro Val Trp Ser Tyr Leu Val Cys Pro
1               5                   10                  15

<210>  60
<211>  25
<212>  PRT
<213>  Phanerochaete chrysosporium

<400>  60
Arg Ile Ser Trp Phe Phe Ser Ala Leu Thr Pro Leu Leu Leu Ala Asp
1               5                   10                  15

Pro Cys Arg His Ile Phe Lys Ser Ala
            20                  25

<210>  61
<211>  39
<212>  PRT
<213>  Phanerochaete chrysosporium

<400>  61
Trp Lys Ala Tyr Met Pro Met Arg Arg Ile Phe Ile Thr Ser Leu Ala
1               5                   10                  15

Arg Asp Glu Leu Leu Ser Arg His Trp Ser Trp Phe Thr Asn Tyr Arg
            20                  25                  30

Asp Ala Ala Glu Val Tyr Met
        35

<210>  62
<211>  24
<212>  PRT
<213>  Phanerochaete chrysosporium

<400>  62
Ala Ala Glu Ser Ser Pro Pro Ser Asn Asn His Arg Asp Asp Val Gln
1               5                   10                  15

Gln Ser Leu Pro Gln Ala Ala Leu
            20

<210>  63
<211>  20
<212>  PRT
<213>  Phanerochaete chrysosporium

<400>  63
Ala Arg Ala Ala Cys Arg Ser Pro Ser Leu Pro Ala Pro Thr Arg Ala
1               5                   10                  15

Gly Arg Thr Ser
            20

<210>  64
<211>  393
<212>  PRT
<213>  Phanerochaete chrysosporium

<400>  64
Trp Leu Leu Gly Arg Gly Ile Pro Leu Ser Leu Arg Leu Gln Gln Met
1               5                   10                  15

Pro Gln His His Trp Leu Trp Thr Arg His Val Arg His Glu Glu Arg
            20                  25                  30

His Pro Asp Val Cys Gln Pro Ile Arg Asn Tyr Gln Gln Ser Val Ser
        35                  40                  45

Pro His Ile Phe Phe Tyr Glu Leu Arg Trp Tyr Asn Leu Ser Ser Arg
    50                  55                  60

Ser Ser Ser Trp Thr Pro Val Ser Leu Ala Lys Leu Asp Phe Pro Val
65                  70                  75                  80

Ala Met His Tyr Ala Asp Ile Thr Lys Asn Gly Phe Asn Asp Gly Arg
                85                  90                  95

Cys Ile Phe Phe Phe Phe Cys Tyr Ile Ser Cys Phe Ala Asn His Arg
            100                 105                 110

Thr Val Ile Ile Thr Asp Gln Tyr Gly Ser Ser Met Asp Asp Ile Trp
        115                 120                 125

Ala Tyr Gly Gly Arg Val Ser Trp Leu Glu Asn Pro Gly Glu Leu Arg
    130                 135                 140

Asp Asn Trp Thr Met Arg Thr Ile Gly His Ser Pro Gly Met His Arg
145                 150                 155                 160

Leu Lys Ala Gly His Phe Thr Arg Thr Asp Arg Val Gln Val Val Ala
                165                 170                 175

Val Pro Ile Val Val Ala Ser Ser Asp Leu Thr Thr Pro Ala Asp Val
            180                 185                 190

Ile Ile Phe Thr Ala Pro Asp Asp Pro Arg Ser Glu Gln Leu Trp Gln
        195                 200                 205

Arg Asp Val Val Gly Thr Arg His Leu Val His Glu Val Ala Ile Val
    210                 215                 220

Pro Ala Ala Glu Thr Asp Gly Glu Met Arg Phe Asp Gln Ile Ile Leu
225                 230                 235                 240

Ala Gly Arg Asp Gly Val Asp Cys Leu Trp Tyr Asp Gly Ala Arg Trp
                245                 250                 255

Gln Lys His Leu Val Gly Thr Gly Leu Pro Glu Glu Arg Gly Asp Pro
            260                 265                 270

Tyr Trp Gly Ala Gly Ser Ala Ala Val Gly Arg Val Gly Asp Asp Tyr
        275                 280                 285

Ala Gly Tyr Ile Cys Ser Ala Glu Val Gly Phe Gly Ser Ile Ile Phe
    290                 295                 300

Arg Arg Ser Leu Thr Gly Ile Phe Ala Gly Ile Pro Arg Gln Tyr Arg
305                 310                 315                 320

Leu Gly Leu Tyr Lys Ala Arg Trp Leu Thr Asp Gly His Arg Pro Arg
                325                 330                 335

Arg Val Asp Glu Thr Cys Ala Arg Arg Leu Arg Ala Thr Gln Arg Glu
            340                 345                 350

Ala His Arg Glu His Ser Pro Gly Arg Leu Arg Gly His Arg Trp Arg
        355                 360                 365

Arg Gly Arg Arg Ile Ser Arg Ser His Asp Gly Arg Arg Ser Ser Gly
    370                 375                 380

Leu Pro Glu Asp Arg Arg Leu Val Leu
385                 390

<210>  65
<211>  23
<212>  PRT

<213>  Phanerochaete chrysosporium

<400>  65
Val Asn Phe Gly Val Phe Asn Asp Thr Asp Ala Asp Cys Ala Leu Trp
1               5                   10                  15

Gln Leu Ser Thr Gly Gln Thr
            20

<210>  66
<211>  82
<212>  PRT
<213>  Phanerochaete chrysosporium

<400>  66
Ser Ser Pro Arg Pro Lys Ser Val Val Phe Leu Pro Gly Ala Ser Gln
1               5                   10                  15

Gln Arg Thr Ser Thr Arg Arg Ala Pro Lys Trp Cys Val Phe Cys Pro
            20                  25                  30

Ala Leu Thr Met Arg Gln Asn Ile His Thr Asp Leu Ser Arg Thr Leu
        35                  40                  45

Pro Pro Ser Leu Thr Leu Phe Leu Asp Ile Leu Ser Pro Pro Thr Arg
    50                  55                  60

Pro Ser Thr Ser Ser Ser Pro Pro Ala Phe Leu Pro Ser Gly Leu Thr
65                  70                  75                  80

Lys Arg

<210>  67
<211>  45
<212>  PRT
<213>  Phanerochaete chrysosporium

<400>  67
Cys Ser Gly Trp Ser Ala Gln Asp Arg Arg Ala Ser Arg Pro Arg Trp
1               5                   10                  15

Ser Ser Leu Thr Ser Arg Glu Arg Ser Leu Arg Leu Ser Cys Cys Arg
            20                  25                  30

Pro Ser His Ala Ser Met Ser Asn Ala Met Cys Pro Val
        35                  40                  45

<210>  68
<211>  29
<212>  PRT
<213>  Phanerochaete chrysosporium

<400>  68
Arg Ser Trp Pro Gly Gln Ser Val Gly Pro Thr Arg Thr Gly Ser Met
1               5                   10                  15

Asn Ala Cys Leu Gln Arg Ala His Ser Ala Ala Arg Ala
            20                  25

<210>  69
<211>  52
<212>  PRT
<213>  Phanerochaete chrysosporium

<400>  69
Ser Ser Pro Gln Thr Ile Ser Arg Ala Gly Lys Arg Ala Arg Ser Ser
1               5                   10                  15

Ser Ser Thr Ser Pro Arg Ala Pro Gln Ala Gly Arg Arg Ser Val Leu
            20                  25                  30

Trp Thr Asn Leu Trp Arg Thr Thr Cys Ser Pro Arg Thr Ser Pro Ile
        35                  40                  45

Val Phe Ala Arg
    50

<210>  70
<211>  18
<212>  PRT
<213>  Phanerochaete chrysosporium

<400>  70
Ser Ser Pro Gly Tyr Ala Ala Gln Ile Ala Arg Gly Arg Met Ala Ala
1               5                   10                  15

Ser Arg

<210>  71
<211>  8
<212>  PRT
<213>  Phanerochaete chrysosporium

<400>  71
Cys Phe Ser Arg Ser Pro Leu Glu
1               5

<210>  72
<211>  11
<212>  PRT
<213>  Phanerochaete chrysosporium

<400>  72
Pro Ser Ser Leu Thr Leu Ala Met Ile Gly Pro
1               5                   10

<210>  73
<211>  13
<212>  PRT
<213>  Phanerochaete chrysosporium

<400>  73
Leu Leu Gln Pro His Arg Leu Pro Arg Gln Leu Cys Gly
1               5                   10


<210>  74
<211>  71
<212>  PRT
<213>  Phanerochaete chrysosporium

<400>  74
Phe Arg Gly Cys Ala Arg Ala Arg Ser Ala Leu Asp Gly Gly His Trp
1               5                   10                  15


Arg Leu Arg Trp Val Pro Gln Pro Arg Arg Ser Val Val Leu Arg Asp
            20                  25                  30


Pro Cys Leu His Arg Glu Arg His Arg Ser Arg Arg Asp Ala Leu Gly
        35                  40                  45


Asn Arg Ser Arg Cys Gln Phe Gln Pro Arg Gln Pro Glu Pro Arg Gly
    50                  55                  60


His Gly Ala Asp Cys Arg Ala
65                  70


<210>  75
<211>  12
<212>  PRT
<213>  Phanerochaete chrysosporium

<400>  75
His Ala Arg Ala Arg Pro Thr Leu Ala His Ala Ser
1               5                   10


<210>  76
<211>  8
<212>  PRT
<213>  Phanerochaete chrysosporium

<400>  76
Trp Thr Pro Ala Pro Ala His Glu
1               5


<210>  77
<211>  12
<212>  PRT
<213>  Phanerochaete chrysosporium

<400>  77
His His Arg Leu Pro Met Ala Trp Cys Val His Asp

1               5                   10

<210>  78
<211>  143
<212>  PRT
<213>  Phanerochaete chrysosporium

<400>  78
Leu Arg Arg Thr Ser Ala Leu Thr Arg Leu Cys Val Thr Ser Leu Ala
1               5                   10                  15

Gly Gly Arg Arg Gln Pro Gln Pro Ala Gly Val Arg Arg Leu Gly Cys
            20                  25                  30

Val Arg Val Pro Arg Val Arg Asn Val Leu Asp Ser Ser Ala Ser Ala
        35                  40                  45

Arg Thr Arg Ala Arg Glu Val Arg Asn Pro Val Trp Arg Pro Ser Pro
    50                  55                  60

Asp Arg Ile Ala Cys Pro Ser Glu Glu Arg Cys His Arg Arg His Pro
65                  70                  75                  80

Arg Ser Arg Ala Pro Arg Pro Arg Trp Arg Pro Val Ala Ala Gly Gly
                85                  90                  95

Glu Ser Tyr Leu Phe Cys Ala Arg Thr Tyr Lys Leu Thr Trp Thr Leu
            100                 105                 110

Ser Val Glu Tyr Leu Ser Cys Ser Arg His Gly His Val Arg Asp Arg
        115                 120                 125

Ala Arg Gln Asp Gly Phe Ala Cys Leu Cys Ser Leu Ala Ala Arg
    130                 135                 140

<210>  79
<211>  47
<212>  PRT
<213>  Phanerochaete chrysosporium

<400>  79
Glu Ser Ala Cys Arg Arg His Ala Leu Ser Cys Cys His Gly Ser Tyr
1               5                   10                  15

Val Thr Val Gly Arg His Glu Glu His Gln Gly Ala Asp Tyr Val Arg
            20                  25                  30

Asn Pro Val Gly Ile Ala Ala Val Val Met Leu Ser Ala Cys Met
        35                  40                  45

<210>  80

<211>  14
<212>  PRT
<213>  Phanerochaete chrysosporium

<400>  80
Leu Pro Ser Pro Gly Gly Ala Arg Pro Trp Pro Ala Leu Pro
1               5                   10

<210>  81
<211>  16
<212>  PRT
<213>  Phanerochaete chrysosporium

<400>  81
Arg Phe Arg Tyr Thr Pro Pro Thr Gly Ser Arg Arg Asp Ser Arg Thr
1               5                   10                  15

<210>  82
<211>  55
<212>  PRT
<213>  Phanerochaete chrysosporium

<400>  82
Thr Ala Ile Pro Val Gln Val Leu Ser His Val Phe Thr Leu Tyr Pro
1               5                   10                  15

Leu Arg Ser Ser Cys Arg Gly Thr Ala Leu Ser Phe Arg Arg Gly Leu
            20                  25                  30

Leu Phe Leu Pro Thr Glu Lys Val Ser Trp Lys Thr Gly Ser Trp Asn
        35                  40                  45

Val Ser Leu Tyr Asn Ser Val
    50                  55

<210>  83
<211>  26
<212>  PRT
<213>  Phanerochaete chrysosporium

<400>  83
Ser Asn Lys Ala Met Cys Tyr Arg Gln Trp Ser Ser Asn Asp Ser Thr
1               5                   10                  15

Cys Leu Lys Arg Met Arg Gly Tyr Val Thr
            20                  25

<210>  84
<211>  13
<212>  PRT
<213>  Phanerochaete chrysosporium

<400>  84
Gly Val Arg Ala Arg Leu Pro Gln Thr Gln Pro Ala Ala
1               5                   10

## Description

### FIELD OF THE INVENTION

The present invention relates to sequences. In particular, the present invention relates to the amino acid sequence of pyranosone dehydratase, and nucleic acid sequences encoding therefor.

### TECHNICAL BACKGROUND AND PRIOR ART

It is well documented in the literature that glucose can be oxidized by pyranose 2-oxidase (EC 1.1.3.10, P2O) to form glucosone (D-arabino-hexos-2-ulose), which in turn can be converted to cortalcerone by pyranosone dehydratase (PD) [Koths, K.; Halenbeck, R.; Moreland, M. (1992), Carbohydr Res. Vol. 232 No. 1, PP. 59-75; Gabriel, J.; Volc, J.; Sedmera, P.; Daniel, G.; Kubatova, E. (1993), Arch. Microbi., 160:27-34]. Both P20 and PD have been purified in fungi and P20 has been cloned. PD has been purified from *Polyporus obtusus* by Koths et al (1992), and from *Phanerochaete chrysosporium* by Gabriel et al (1993). However, to date, there has been no amino acid or nucleotide sequence characterisation of PD.

It has been established in the art that starch can be converted to 1,5-anhydro-D-fructose (AF) [S. Yu and J. Marcussen, Recent Advances in Carbohydrate Bioengineering; Gilbert, H. J.; Davies, G. J; Henrissat B.; Svensson, B., Eds.; Royal Society of Chemistry (RS.C) Press, 1999. 242-250]. It has further been shown that several fungal and red algal extracts can convert AF to microthecin possibly enzymatically, but the enzymes involved have not been isolated, purified or characterized [Baute, M-A.; Deffieux, G.; Baute, R. (1986), Phytochemistry (Oxf) vol. 25:1472-1473; Broberg, A., Kenne, L., and Pedersén, M. (1996), Phytochemistry (oxf). 41: 151-154]. To date, there has been no suggestion that PD could play a role in the conversion of AF to microthecin.

It has also been documented that ascopyrone P (APP) can be produced from AF [ Baute, M-A.; Deffieux, G.; Vercauteren, J.; Baute, R.; Badoc, A. (1993), Phytochemistry (oxf) vol. 33 no. 1, 41-45]. Again, there has been no evidence to suggest the involvement of PD in this process.

### SUMMARY OF THE INVENTION

In a broad aspect the invention relates to characterisation of the amino acid sequence and nucleotide sequence encoding for pyranosone dehydratase.

Further aspects of the invention relate to previously undisclosed uses of pyranosone dehydratase which include the conversion AF to microthecin and APP, and the conversion of glucosone to cortalcerone.

Aspects of the present invention are presented in the claims and in the following commentary.

In brief, some aspects of the present invention relate to:
1. A novel amino acid sequence
2. A novel nucleotide sequence
3. Methods of preparing said amino acid sequence
4. Methods of preparing said nucleotide sequence
5. Expression systems comprising said nucleotide sequence
6. Methods of expressing said nucleotide sequence
7. Transformed hosts/host cells comprising said nucleotide sequence
8. Uses of said amino acid sequence
9. Uses of said nucleotide sequence

As used with reference to the present invention, the terms "expression", "expresses", "expressed" and "expressable" are synonymous with the respective terms "transcription", "transcribes", "transcribed" and "transcribable".

Other aspects concerning the nucleotide sequence of the present invention include: a construct comprising the sequences of the present invention; a vector comprising the sequences of the present invention; a plasmid comprising the sequences of present invention; a transformed cell comprising the sequences of the present invention; a transformed tissue comprising the sequences of the present invention; a transformed organ comprising the sequences of the present invention; a transformed host comprising the sequences of the present invention; a transformed organism comprising the sequences of the present invention. The present invention also encompasses methods of expressing the nucleotide sequence using the same, such as expression in a host plant cell; including methods for transferring same.

For ease of reference, these and further aspects of the present invention are now discussed under appropriate section headings. However, the teachings under each section are not necessarily limited to each particular section.

### DETAILED DISCLOSURE OF INVENTION

In one aspect the invention relates to an isolated polypeptide comprising at least one amino acid sequence selected from the following:
(i) KPHCEPEQPAALPLFQPQLVQGGRPDXYWVEAFPFRSDSSK;
(ii) SDIQMFVNPYATTNNQSSXWTPVSLAKLDFPVAMHYADITK;
(iii) VSWLENPGELR;
(iv) DGVDCLWYDGAR;
(v) PAGSPTGIVRAEWTRHVLDVFGXLXXK;
(vi) HTGSIHQVVCADIDGDGEDEFLVAMMGADPPDFQRTGVWCYK;
(vii) TEMEFLDVAGK;
(viii) KLTLVVLPPFARLDVERNVSGVK;
(ix) SMDELVAHNLFPAYVPDSVR;
(x) NDATDGTPVLALLDLDGGPSPQAWNISHVPPGTDMYEIAHAK;
(xi) TGSLVCARWPPVK;
(xii) NQRVAGTHSPAAMGLTSRWAVTK;
(xiii) GQITFRLPEAPDHGPLFLSVSAIRHQ;
(xiv) KPHXEPEQPAALPLFQPQLVV(Q)GGRPDXY;
where X is an unknown amino acid residue; or a variant, homologue or derivative thereof.

In a yet further aspect, the invention relates to a nucleotide sequence selected from:
(a) the nucleotide sequence encoding for the above amino acid sequence;
(b) a nucleotide sequence that is a variant, homologue, derivative or fragment of the nucleotide sequence of (a);
(c) a nucleotide sequence that is the complement of the nucleotide sequence of (a)
(d) a nucleotide sequence that is the complement of a variant, homologue, derivative or fragment of the nucleotide sequence of (a);
(e) a nucleotide sequence that is capable of hybridising to the nucleotide sequence of (a);
(f) a nucleotide sequence that is capable of hybridising to a variant, homologue, derivative or fragment of the nucleotide sequence of (a);
(g) a nucleotide sequence that is the complement of a nucleotide sequence that is capable of hybridising to the nucleotide sequence of (a);
(h) a nucleotide sequence that is the complement of a nucleotide sequence that is capable of hybridising to a variant, homologue, derivative or fragment of the nucleotide sequence of (a);
(i) a nucleotide sequence that is capable of hybridising to the complement of the nucleotide sequence of (a);
(j) a nucleotide sequence that is capable of hybridising to the complement of a variant, homologue, derivative or fragment of the nucleotide sequence of (a);
(k) a nucleotide sequence comprising any one of (a), (b), (c), (d), (e), (f), (g), (h), (i), and/or (j).

Another aspect of the present invention includes an isolated nucleotide sequence according to the present invention.

### PREFERABLE ASPECTS

In one preferred embodiment, the invention relates to an isolated polypeptide comprising at least one amino acid sequence selected from (i) to (xiii) below:
(i) KPHCEPEQPAALPLFQPQLVQGGRPDXYWVEAFPFRSDSSK or KPHXEPEQPAALPLFQPQLVV(Q)GGRPDXY;
(ii) SDIQMFVNPYATTNNQSSXWTPVSLAKLDFPVAMHYADITK;
(iii) VSWLENPGELR;
(iv) DGVDCLWYDGAR;
(v) PAGSPTGIVRAEWTRHVLDVFGXLXXK;
(vi) HTGSIHQVVCADIDGDGEDEFLVAMMGADPPDFQRTGVWCYK;
(vii) TEMEFLDVAGK;
(viii) KLTLVVLPPFARLDVERNVSGVK;
(ix) SMDELVAHNLFPAYVPDSVR;
(x) NDATDGTPVLALLDLDGGPSPQAWNISHVPPGTDMYEIAHAK;
(xi) TGSLVCARWPPVK;
(xii) NQRVAGTHSPAAMGLTSRWAVTK;
(xiii) GQITFRLPEAPDHGPLFLSVSAIRHQ;
where X is an unknown amino acid residue; or a variant, homologue or derivative thereof.

In one preferred embodiment, said polypeptide comprises one sequence selected from sequences (i) to (xiii) above.

In another preferred embodiment, said polypeptide comprises two sequences selected from sequences (i) to (xiii) above.

In another preferred embodiment, said polypeptide comprises three sequences selected from sequences (i) to (xiii) above.

In another preferred embodiment, said polypeptide comprises four sequences selected from sequences (i) to (xiii) above.

In another preferred embodiment, said polypeptide comprises five sequences selected from sequences (i) to (xiii) above.

In another preferred embodiment, said polypeptide comprises six sequences selected from sequences (i) to (xiii) above.

In another preferred embodiment, said polypeptide comprises seven sequences selected from sequences (i) to (xiii) above.

In another preferred embodiment, said polypeptide comprises eight sequences selected from sequences (i) to (xiii) above.

In another preferred embodiment, said polypeptide comprises nine sequences selected from sequences (i) to (xiii) above.

In another preferred embodiment, said polypeptide comprises ten sequences selected from sequences (i) to (xiii) above.

In another preferred embodiment, said polypeptide comprises eleven sequences selected from sequences (i) to (xiii) above.

In another preferred embodiment, said polypeptide comprises twelve sequences selected from sequences (i) to (xiii) above.

In another preferred embodiment, said polypeptide comprises thirteen sequences selected from sequences (i) to (xiii) above.

Preferably, the polypeptide of the invention has pyranosone dehydratase activity.

One preferred embodiment relates to a polypeptide that is immunologically reactive with an antibody raised against a purified amino acid sequence according to the invention.

Another aspect relates to an isolated polynucleotide sequence encoding a polypeptide of the invention, or a variant, homologue, fragment or derivative thereof.

Preferably, the isolated polynucleotide is selected from:
(i) a polynucleotide comprising the nucleotide sequence of SEQ ID No. 1 or the complement thereof;
(ii) a polynucleotide comprising a nucleotide sequence capable of hybridising to the nucleotide sequence of SEQ ID No. 1, or a fragment thereof;
(iii) a polynucleotide comprising a nucleotide sequence capable of hybridising to the complement of the nucleotide sequence of SEQ ID. No. 1; and
(iv) a polynucleotide comprising a polynucleotide sequence which is degenerate as a result of the genetic code to the polynucleotide of SEQ ID No. 1.

Preferably, the nucleotide sequence is obtainable from *Phanerochaete chrysosporium, Polyporus obtusus* or *Corticium caeruleum.*

In one preferred embodiment, the nucleotide sequence is obtainable from the order of *Pezizales,* more preferably from *Aleuria aurantia, Peziza badia, P. succosa, Sarcophaera eximia, Morchella conica, M costata, M elata, M esculenta, M esculenta var. rotunda, M hortensis* or *Gyromitra infula.*

In another preferred embodiment, the nucleotide sequence is obtainable from the order *of Auriculariales,* more preferably from *Auricularia mesenterica.*

In another preferred embodiment, the nucleotide sequence is obtainable from the order of *Aphyllophorales,* more preferably from *Pulcherricium caeruleum, Peniophora quercina, Phanerochaete sordida, Vuilleminia comedens, Stereum gausapatum, S. sanguinolentum, Lopharia spadicea, Sparassis laminosa, Boletopsis subsquamosa, Bjerkandera adusta, Trichaptum biformis, Cerrena unicolor, Pycnoporus cinnabarinus, P. sanguineus, Junghunia nitida, Ramaria flava, Clavulinopsis helvola, C. helvola var. geoglossoides* or *V. pulchra.*

In another preferred embodiment, the nucleotide sequence is obtainable from the order of *Agaricales,* more preferably from *Clitocybe cyathiformis, C. dicolor, C. gibba, C. odora, Lepista caespitosa, L inversa, L. luscina, L. nebularis, Mycena seynii, Pleurocybella porrigens, Marasmius oreales* or *Inocybe pyriodora.*

In another preferred embodiment, the nucleotide sequence is obtainable from the order *Gracilariales,* more preferably from *Gracilaria varrucosa, Gracilaria tenuistipitata, Gracilariopsis sp,* or *Gracilariopsis lemaneiformis.*

In another preferred embodiment, the nucleotide sequence is obtainable from the order of Melanosporaceae more preferably from *Melanospora ornata, Microthecium compressum, Microthecium sobelii.*

Another aspect of the invention relates to an isolated polynucleotide which is selected from:
(i) a polynucleotide comprising the nucleotide sequence of SEQ ID No. 1 or the complement thereof;
(ii) a polynucleotide comprising a nucleotide sequence capable of hybridising to the nucleotide sequence of SEQ ID No. 1, or a fragment thereof;
(iii) a polynucleotide comprising a nucleotide sequence capable of hybridising to the complement of the nucleotide sequence of SEQ ID. No. 1; and
(iv) a polynucleotide comprising a polynucleotide sequence which is degenerate as a result of the genetic code to the polynucleotide of SEQ ID No. 1.

Preferably, the nucleotide sequence is operably linked to a promoter.

Another aspect of the invention relates to a construct comprising the above polynucleotide sequence.

Yet another aspect relates to a vector comprising the above polynucleotide sequence.

A further aspect relates to a host cell into which has been incorporated the polynucleotide sequence of the invention.

Another aspect relates to an expression vector comprising a polynucleotide sequence of the invention operably linked to a regulatory sequence capable of directing expression of said polynucleotide in a host cell.

A further aspect relates to an isolated polypeptide encoded by the polynucleotide sequence of SEQ ID NO.1, or a variant, homologue, fragment or derivative thereof.

In a preferred embodiment, said isolated polypeptide has up to 7 amino acids removed from the N-terminus.

More preferably, the isolated polypeptide has at least 75% identity to a polypeptide sequence encoded by SEQ ID NO.1.

Yet another aspect relates to an antibody capable of binding a polypeptide according to the invention.

Another aspect relates to method of preparing an amino acid sequence of the invention wherein said process comprises expressing the nucleotide sequence of the invention, and optionally isolating and/or purifying the same. Preferably, the nucleotide sequence of the invention is expressed in an environment which is free from the substrates of the expressed enzyme, for example, in an environment which is free from 1,5-anhydrofructose.

A further aspect relates to a process for preparing microthecin using the amino acid sequence of the invention, or the expression product of the nucleotide sequence of the invention.

Yet another aspect relates to a process for preparing ascopyrone P using the amino acid sequence of the invention, or the expression product of the nucleotide sequence of the invention.

Preferably, the process comprises reacting said amino acid sequence or said expression product of the nucleotide sequence with 1,5-anhydro-D-fructose.

Even more preferably, the process further comprises the use of APP synthase.

In a particularly preferred embodiment, the process comprises reacting APP synthase and said amino acid sequence or said expression product of the nucleotide sequence with 1,5-anhydro-D-fructose.

In an alternative preferred embodiment, said process for making microthecin comprises contacting a polypeptide according to the invention with glucan lyase and dextrins starch.

A further aspect of the invention relates to a process for preparing cortalcerone using the amino acid sequence of the invention or the expression product of the nucleotide sequence of the invention.

Preferably, said process comprises reacting the amino acid sequence or the expression product of the nucleotide sequence with glucosone.

In an alternative preferred embodiment, said process for making microthecin comprises reacting a polypeptide of the invention with glucose and pyranose 2-oxidase.

Another aspect of the invention relates to a process for preparing microthecin comprising reacting pyranosone dehydratase with 1,5-anhydro-D-fructose.

Yet another aspect of the invention relates to a process for preparing ascopyrone P comprising reacting pyranosone dehydratase and APP synthase with 1,5-anhydro-D-fructose.

One aspect of the invention relates to a process for preparing microthecin comprising reacting pyranosone dehydratase with glucose and dextrins starch.

Another aspect of the invention relates to process for preparing cortalcerone comprising reacting pyranosone dehydratase with glucosone.

Yet another aspect of the invention relates to a process for preparing cortalcerone comprising reacting pyranosone dehydratase with glucose and pyranose 2-oxidase.

Another aspect of the invention relates to the use of microthecin for preventing and/or inhibiting the growth of, and/or killing, microorganisms in a material.

An alternative aspect of the invention relates to the use of cortalcerone for preventing and/or inhibiting the growth of, and/or killing, microorganisms in a material.

The invention also relates to the use of one or more of microthecin, cortalcerone, or derivatives or isomers thereof, for preventing and/or inhibiting the growth of, and/or killing, microorganisms in a material.

Preferably, the material is a foodstuff.

Preferably the microorganisms against which cortalcerone and/or microthecin are active are plant fungal pathogens.

Preferably the microorganisms against which cortalcerone and/or microthecin are active are selected from microorganisms selected from the orders *Rhizoctonia, Pythium, Aphanomyces* and *Cercospora.*

Preferably the microorganisms against which cortalcerone and/or microthecin are active are selected from microorganisms selected from *Rhizoctonia solani, Pythium ultimum, Aphanomyces cochlioides* and *Cercospora beticola.*

A further aspect of the invention relates to the use of microthecin, cortalcerone, or derivatives or isomers thereof, in preventing and/or inhibiting the growth of, and/or killing the pathogen *Aphanomyces.*

Preferably, the pathogen is *Aphanomyces cochlioides.*

In a preferred embodiment, the derivative of microthecin is 2-furyl-hydroxymethyl-ketone or 4-deoxy-*glycero*-hexo-2,3-diluose.

In a preferred embodiment, the derivative of cortalcerone is 2-furylglyoxal.

In a particularly preferred embodiment, the microthecin, cortalcerone, or derivatives or isomers thereof, is used in the treatment of plants or plant seeds, even more preferably, in the treatment of sugar beet seeds, pea plants or pea plant seeds.

In another aspect, the invention relates to the use of microthecin, cortalcerone, or derivatives or isomers thereof, as plant or seed protectants.

Yet another aspect of the invention relates to the use of microthecin, cortalcerone, or derivatives or isomers thereof, as plant growth regulators.

### ADVANTAGES

The present invention provides previously undisclosed amino acid and nucleotide sequence information in respect of pyranosone dehydratase.

The invention further relates to the use of pyranosone dehydratase in the conversion of AF to APP and microthecin, and in the production of cortalcerone. To date, there has been no teaching or suggestion in the art that PD is involved in, or capable of effecting, either of these conversions.

The present invention could thus facilitate the large scale production of microthecin, APP and cortalcerone. The invention further teaches that microthecin and cortalcerone have useful applications as antimicrobial agents, more particularly in foodstuffs.

### ASSAY

The following assay may be used to characterise and identify actual and putative amino acid sequences according to the present invention.

### ISOLATED

In one aspect, preferably the sequence is in an isolated form. The term "isolated" means that the sequence is not in its naural environment (i.e. as found in nature). Typically the term "isolated" means that the sequence is at least substantially free from at least one other compnent with which the sequence is naturally associated in nature and as found in nature. Here, the sequence may be separated from at least one other component with which it is naturally associated.

### PURIFIED

In one aspect, preferably the sequence is in a purified form. The term "purified" also means that the sequence is not in its naural environment (i.e. as found in nature). Typically the term "purified" means that the sequence is at least substantially separated from at least one other compnent with which the sequence is naturally associated in nature and as found in nature.

### NUCLEOTIDE SEQUENCE

The present invention encompasses nucleotide sequences encoding enzymes having the specific properties as defined herein. The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be double-stranded or single-stranded whether representing the sense or antisense strand.

The term "nucleotide sequence" in relation to the present invention includes genomic DNA, cDNA, synthetic DNA, and RNA. Preferably it means DNA, more preferably cDNA for the coding sequence of the present invention.

In a preferred embodiment, the nucleotide sequence *per se* of the present invention does not cover the native nucleotide sequence according to the present invention in its natural environment when it is linked to its naturally associated sequence(s) that is/are also in its/their natural environment. For ease of reference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment. However, the amino acid sequence of the present invention can be isolated and/or purified post expression of a nucleotide sequence in its native organism. Preferably, however, the amino acid sequence of the present invention may be expressed by a nucleotide sequence in its native organism but wherein the nucleotide sequence is not under the control of the promoter with which it is naturally associated within that organism.

Typically, the nucleotide sequence of the present invention is prepared using recombinant DNA techniques (i.e. recombinant DNA). However, in an alternative embodiment of the invention, the nucleotide sequence could be synthesised, in whole or in part, using chemical methods well known in the art (see Caruthers MH et al (1980) Nuc Acids Res Symp Ser 215-23 and Horn T et al (1980) Nuc Acids Res Symp Ser 225-232).

### PREPARATION OF THE NUCLEOTIDE SEQUENCE

A nucleotide sequence encoding either an enzyme which has the specific properties as defined herein or an enzyme which is suitable for modification may be identified and/or isolated and/or purified from any cell or organism producing said enzyme. Various methods are well known within the art for the identification and/or isolation and/or purification of nucleotide sequences. By way of example, PCR amplification techniques to prepare more of a sequence may be used once a suitable sequence has been identified and/or isolated and/or purified.

By way of further example, a genomic DNA and/or cDNA library may be constructed using chromosomal DNA or messenger RNA from the organism producing the enzyme. If the amino acid sequence of the enzyme is known, labelled oligonucleotide probes may be synthesised and used to identify enzyme-encoding clones from the genomic library prepared from the organism. Alternatively, a labelled oligonucleotide probe containing sequences homologous to another known enzyme gene could be used to identify enzyme-encoding clones. In the latter case, hybridisation and washing conditions of lower stringency are used.

Alternatively, enzyme-encoding clones could be identified by inserting fragments of genomic DNA into an expression vector, such as a plasmid, transforming enzyme-negative bacteria with the resulting genomic DNA library, and then plating the transformed bacteria onto agar containing a substrate for enzyme (i.e. maltose), thereby allowing clones expressing the enzyme to be identified.

In a yet further alternative, the nucleotide sequence encoding the enzyme may be prepared synthetically by established standard methods, e.g. the phosphoroamidite method described by Beucage S.L. et al (1981) Tetrahedron Letters 22, p 1859-1869, or the method described by Matthes et al (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al (Science (1988) 239, pp 487-491).

### AMINO ACID SEQUENCES

The present invention also encompasses amino acid sequences of enzymes having the specific properties as defined herein.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The amino acid sequence may be prepared/isolated from a suitable source, or it may be made synthetically or it may be prepared by use of recombinant DNA techniques.

The enzyme of the present invention may be used in conjunction with other enzymes. Thus the present invention also covers a combination of enzymes wherein the combination comprises the enzyme of the present invention and another enzyme, which may be another enzyme according to the present invention. This aspect is discussed in a later section.

Preferably the enzyme is not a native enzyme. In this regard, the term "native enzyme" means an entire enzyme that is in its native environment and when it has been expressed by its native nucleotide sequence.

### VARIANTS/HOMOLOGUES/DERIVATIVES

The present invention also encompasses the use of variants, homologues and derivatives of any amino acid sequence of an enzyme of the present invention or of any nucleotide sequence encoding such an enzyme. Here, the term "homologue" means an entity having a certain homology with the subject amino acid sequences and the subject nucleotide sequences. Here, the term "homology" can be equated with "identity".

In the present context, an homologous sequence is taken to include an amino acid sequence which may be at least 75, 80, 85 or 90% identical, preferably at least 95, 96, 97, 98 or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, an homologous sequence is taken to include a nucleotide sequence which may be at least 40, 50, 60, 70, 75, 80, 85 or 90% identical, preferably at least 95, 96, 97, 98 or 99% identical to a nucleotide sequence encoding an enzyme of the present invention (the subject sequence). Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% Homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (Devereux et al 1984 Nuc. Acids Research 12 p387). Examples of other software than can perform sequence comparisons include, but are not limited to, the BLAST package (see Ausubel et al 1999 Short Protocols in Molecular Biology, 4th Ed - Chapter 18), FASTA (Altschul et al 1990 J. Mol. Biol. 403-410) and the GENEWORKS suite of comparison tools. Both BLAST and FASTA are available for offline and online searching (see Ausubel *et al* 1999, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | IL V |
| | Polar - uncharged | C S T M |
| | | N Q |
| | Polar - charged | D E |
| | | K R |
| AROMATIC | | H F W Y |

The present invention also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, β-alanine*, L-α-amino butyric acid*, L-γ-amino butyric acid*, L-α-amino isobutyric acid*, L-ε-amino caproic acid^{#}, 7-amino heptanoic acid*, L-methionine sulfone^{#*}, L-norleucine*, L-norvaline*, p-nitro-L-phenylalanine*, L-hydroxyproline^{#}, L-thioproline*, methyl derivatives of phenylalanine (Phe) such as 4-methyl-Phe*, pentamethyl-Phe*, L-Phe (4-amino)^{#}, L-Tyr (methyl)*, L-Phe (4-isopropyl)*, L-Tic (1,2,3,4-tetrahydroisoquinoline-3-carboxyl acid)*, L-diaminopropionic acid ^{#} and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The nucleotide sequences for use in the present invention may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide sequences of the present invention.

The present invention also encompasses the use of nucleotide sequences that are complementary to the sequences presented herein, or any derivative, fragment or derivative thereof. If the sequence is complementary to a fragment thereof then that sequence can be used as a probe to identify similar coding sequences in other organisms etc.

Polynucleotides which are not 100% homologous to the sequences of the present invention but fall within the scope of the invention can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the invention.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

The present invention also encompasses polynucleotides which have undergone molecular evolution via random processes, selection mutagenesis or in vitro recombination. As a non-limiting example, it is possible to produce numerous site directed or random mutations into a nucleotide sequence, either in vivo or in vitro, and to subsequently screen for improved functionality of the encoded polypeptide by various means. In addition, mutations or natural variants of a polynucleotide sequence can be recombined with either the wildtype or other mutations or natural variants to produce new variants. Such new variants can also be screened for improved functionality of the encoded polypeptide. The production of new preferred variants can be achieved by various methods well established in the art, for example the Error Threshold Mutagenesis (WO 92/18645), oligonucleotide mediated random mutagenesis (US 5,723,323), DNA shuffling (US 5,605,793), exo-mediated gene assembly WO 00/58517. The application of these and similar random directed molecular evolution methods allows the identification and selection of variants of the enzymes of the present invention which have preferred characteristics without any prior knowledge of protein structure or function, and allows the production of non-predictable but beneficial mutations or variants. There are numerous examples of the application of molecular evolution in the art for the optimisation or alteration of enzyme activity, such examples include, but are not limited to one or more of the following: optimised expression and/or activity in a host cell or in vitro, increased enzymatic activity, altered substrate and/or product specificity, increased or decreased enzymatic or structural stability, altered enzymatic activity/specificity in preferred environmental conditions, e.g. temperature, pH, substrate.

Polynucleotides (nucleotide sequences) of the invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein.

Polynucleotides such as DNA polynucleotides and probes according to the invention may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

### BIOLOGICALLY ACTIVE

Preferably, the variant sequences etc. are at least as biologically active as the sequences presented herein.

As used herein "biologically active" refers to a sequence having a similar structural function (but not necessarily to the same degree), and/or similar regulatory function (but not necessarily to the same degree), and/or similar biochemical function (but not necessarily to the same degree) of the naturally occurring sequence.

### ISOZYMES

The polypeptide of the present invention may exist in the form of one or more different isozymes. As used herein, the term "isozyme" encompasses variants of the polypeptide that catalyse the same reaction, but differ from each other in properties such as substrate affinity and maximum rates of enzyme-substrate reaction. Owing to differences in amino acid sequence, isozymes can be distinguished by techniques such as electrophoresis or isoelectric focusing. Different tissues often have different isoenzymes. The sequence differences generally confer different enzyme kinetic parameters that can sometimes be interpreted as fine tuning to the specific requirements of the cell types in which a particular isoenzyme is found.

### ISOFORMS

The present invention also encompasses different isoforms of the polypeptide described herein. The term "isoform" refers to a protein having the same function (namely pyranosone dehydratase activity), which has a similar or identical amino acid sequence, but which is the product of a different gene.

### HYBRIDISATION

The present invention also encompasses sequences that are complementary to the sequences of the present invention or sequences that are capable of hybridising either to the sequences of the present invention or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

The present invention also encompasses the use of nucleotide sequences that are capable of hybridising to the sequences that are complementary to the sequences presented herein, or any derivative, fragment or derivative thereof.

The term "variant" also encompasses sequences that are complementary to sequences that are capable of hybridising to the nucleotide sequences presented herein.

Preferably, the term "variant" encompasses sequences that are complementary to sequences that are capable of hybridising under stringent conditions (e.g. 50°C and 0.2xSSC {1xSSC = 0.15 M NaCl, 0.015 M Na₃citrate pH 7.0}) to the nucleotide sequences presented herein.

More preferably, the term "variant" encompasses sequences that are complementary to sequences that are capable of hybridising under high stringent conditions (e.g. 65°C and 0.1xSSC {1xSSC = 0.15 M NaCl, 0.015 M Na₃citrate pH 7.0}) to the nucleotide sequences presented herein.

The present invention also relates to nucleotide sequences that can hybridise to the nucleotide sequences of the present invention (including complementary sequences of those presented herein).

The present invention also relates to nucleotide sequences that are complementary to sequences that can hybridise to the nucleotide sequences of the present invention (including complementary sequences of those presented herein).

Also included within the scope of the present invention are polynucleotide sequences that are capable of hybridising to the nucleotide sequences presented herein under conditions of intermediate to maximal stringency.

In a preferred aspect, the present invention covers nucleotide sequences that can hybridise to the nucleotide sequence of the present invention, or the complement thereof, under stringent conditions (e.g. 50°C and 0.2xSSC).

In a more preferred aspect, the present invention covers nucleotide sequences that can hybridise to the nucleotide sequence of the present invention, or the complement thereof, under high stringent conditions (e.g. 65°C and 0.1xSSC).

### SITE-DIRECTED MUTAGENESIS

Once an enzyme-encoding nucleotide sequence has been isolated, or a putative enzyme-encoding nucleotide sequence has been identified, it may be desirable to mutate the sequence in order to prepare an enzyme of the present invention.

Mutations may be introduced using synthetic oligonucleotides. These oligonucleotides contain nucleotide sequences flanking the desired mutation sites.

A suitable method is disclosed in Morinaga et al (Biotechnology (1984) 2, p646-649), wherein a single-stranded gap of DNA, the enzyme-encoding sequence, is created in a vector carrying the enzyme gene. The synthetic nucleotide, bearing the desired mutation, is then annealed to a homologous portion of the single-stranded DNA. The remaining gap is then filled in with DNA polymerase I (Klenow fragment) and the construct is ligated using T4 ligase.

US 4,760,025 discloses the introduction of oligonucleotides encoding multiple mutations by performing minor alterations of the cassette. However, an even greater variety of mutations can be introduced at any one time by the above mentioned Morinaga method, because a multitude of oligonucleotides, of various lengths, can be introduced.

Another method of introducing mutations into enzyme-encoding nucleotide sequences is described in Nelson and Long (Analytical Biochemistry (1989), 180, p 147-151). This method involves the 3-step generation of a PCR fragment containing the desired mutation introduced by using a chemically synthesised DNA strand as one of the primers in the PCR reactions. From the PCR-generated fragment, a DNA fragment carrying the mutation may be isolated by cleavage with restriction endonucleases and reinserted into an expression plasmid.

By way of example, Sierks et al (Protein Eng (1989) 2, 621-625 and Protein Eng (1990) 3, 193-198) describes site-directed mutagenesis in *Aspergillus* glucoamylase.

### RECOMBINANT

In one aspect of the present invention the sequence is a recombinant sequence - i.e, a sequence that has been prepared using recombinant DNA techniques.

### SYNTHETIC

In one aspect of the present invention the sequence is a synthetic sequence - i.e, a sequence that has been prepared by *in vitro* chemical or enzymatic synthesis. It includes but is not limited to sequences made with optimal codon usage for host organisms, such as the methylotrophic yeasts *Pichia* and *Hansenula.*

### EXPRESSION OF ENZYMES

The nucleotide sequence for use in the present invention can be incorporated into a recombinant replicable vector. The vector may be used to replicate and express the nucleotide sequence, in enzyme form, in and/or from a compatible host cell. Both homologous and heterologous expression is contemplated.

For homologous expression, preferably the gene of interest or nucleotide sequence of interest is not in its naturally occurring genetic context. In the case where the gene of interest or nucleotide sequence of interest is in its naturally occurring genetic context, preferably expression is driven by means other than or in addition to its naturally occurring expression mechanism; for example, by overexpressing the gene of interest by genetic intervention

Expression may be controlled using control sequences which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters described above.

The enzyme produced by a host recombinant cell by expression of the nucleotide sequence may be secreted or may be contained intracellularly depending on the sequence and/or the vector used. The coding sequences can be designed with signal sequences which direct secretion of the substance coding sequences through a particular prokaryotic or eukaryotic cell membrane.

### EXPRESSION VECTOR

The term "expression vector" means a construct capable of *in vivo* or *in vitro* expression.

Preferably, the expression vector is incorporated in the genome of a suitable host organism. The term "incorporated" preferably covers stable incorporation into the genome.

The host organism can be the same or different to the gene of interest source organism, giving rise to homologous and heterologous expression respectively.

Preferably, the vector of the present invention comprises a construct according to the present invention. Alternatively expressed, preferably the nucleotide sequence of the present invention is present in a vector and wherein the nucleotide sequence is operably linked to regulatory sequences such that the regulatory sequences are capable of providing the expression of the nucleotide sequence by a suitable host organism, i.e. the vector is an expression vector.

The vectors of the present invention may be transformed into a suitable host cell as described below to provide for expression of a polypeptide of the present invention. Thus, in a further aspect the invention provides a process for preparing polypeptides for subsequent use according to the present invention which comprises cultivating a host cell transformed or transfected with an expression vector under conditions to provide for expression by the vector of a coding sequence encoding the polypeptides, and recovering the expressed polypeptides.

The vectors may be for example, plasmid, virus or phage vectors provided with an origin of replication, optionally a promoter for the expression of the said polynucleotide and optionally a regulator of the promoter. The choice of vector will often depend on the host cell into which it is to be introduced.

The vectors of the present invention may contain one or more selectable marker genes. The most suitable selection systems for industrial micro-organisms are those formed by the group of selection markers which do not require a mutation in the host organism. Suitable selection markers may be the *dal* genes from *B. subtilis* or *B. licheniformis,* or one which confers antibiotic resistance such as ampicillin, kanamycin, chloramphenicol or tetracyclin resistance. Alternative selection markers may be the *Aspergillus* selection markers such as amdS, argB, niaD and sC, or a marker giving rise to hygromycin resistance. Examples of other fungal selection markers are the genes for ATP synthetase, subunit 9 (*oli*C)*,* orotidine-5'-phosphate-decarboxylase (*pvr*A)*,* phleomycin and benomyl resistance (benA). Examples of non-fungal selection markers are the bacterial G418 resistance gene (this may also be used in yeast, but not in filamentous fungi), the ampicillin resistance gene *(E. coli),* the neomycin resistance gene *(Bacillus)* and the *E. coli uidA* gene, coding for β-glucuronidase (GUS). Further suitable selection markers include the *dal* genes from *B subtilis* or *B. licheniformis.* Alternatively, the selection may be accomplished by co-transformation (as described in WO91/17243).

Vectors may be used *in vitro,* for example for the production of RNA or used to transfect or transform a host cell.

Thus, nucleotide sequences for use according to the present invention can be incorporated into a recombinant vector (typically a replicable vector), for example a cloning or expression vector. The vector may be used to replicate the nucleic acid in a compatible host cell. Thus in a further embodiment, the invention provides a method of making nucleotide sequences of the present invention by introducing a nucleotide sequence of the present invention into a replicable vector, introducing the vector into a compatible host cell, and growing the host cell under conditions which bring about replication of the vector. The vector may be recovered from the host cell. Suitable host cells are described below in connection with expression vectors.

The procedures used to ligate a DNA construct of the invention encoding an enzyme which has the specific properties as defined herein, and the regulatory sequences, and to insert them into suitable vectors containing the information necessary for replication, are well known to persons skilled in the art (for instance see Sambrook et al Molecular Cloning: A laboratory Manual, 2nd Ed. (1989)).

The vector may further comprise a nucleotide sequence enabling the vector to replicate in the host cell in question. Examples of such sequences are the origins of replication of plasmids pUC 19, pACYC177, pUB110, pE 194, pAMB 1 and pIJ702.

The expression vector typically includes the components of a cloning vector, such as, for example, an element that permits autonomous replication of the vector in the selected host organism and one or more phenotypically detectable markers for selection purposes. The expression vector normally comprises control nucleotide sequences encoding a promoter, operator, ribosome binding site, translation initiation signal and optionally, a repressor gene or one or more activator genes. Additionally, the expression vector may comprise a sequence coding for an amino acid sequence capable of targeting the amino acid sequence to a host cell organelle such as a peroxisome or to a particular host cell compartment. In the present context, the term "expression signal" includes any of the above control sequences, repressor or activator sequences. For expression under the direction of control sequences, the nucleotide sequence is operably linked to the control sequences in proper manner with respect to expression.

### REGULATORY SEQUENCES

In some applications, the nucleotide sequence for use in the present invention is operably linked to a regulatory sequence which is capable of providing for the expression of the nucleotide sequence, such as by the chosen host cell. By way of example, the present invention covers a vector comprising the nucleotide sequence of the present invention operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under condition compatible with the control sequences.

The term "regulatory sequences" includes promoters and enhancers and other expression regulation signals.

The term "promoter" is used in the normal sense of the art, e.g. an RNA polymerase binding site.

Enhanced expression of the nucleotide sequence encoding the enzyme of the present invention may also be achieved by the selection of heterologous regulatory regions, e.g. promoter, secretion leader and terminator regions, which serve to increase expression and, if desired, secretion levels of the protein of interest from the chosen expression host and/or to provide for the inducible control of the expression of the enzyme of the present invention. In eukaryotes, polyadenylation sequences may be operably connected to the nucleotide sequence encoding the enzyme.

Preferably, the nucleotide sequence of the present invention may be operably linked to at least a promoter.

Aside from the promoter native to the gene encoding the nucleotide sequence of the present invention, other promoters may be used to direct expression of the polypeptide of the present invention. The promoter may be selected for its efficiency in directing the expression of the nucleotide sequence of the present invention in the desired expression host.

In another embodiment, a constitutive promoter may be selected to direct the expression of the desired nucleotide sequence of the present invention. Such an expression construct may provide additional advantages since it circumvents the need to culture the expression hosts on a medium containing an inducing substrate.

Examples of suitable promoters for directing the transcription of the nucleotide sequence in a bacterial host include the promoter of the *lac* operon of *E. coli,* the *Streptomyces coelicolor* agarase gene *dagA* promoters, the promoters of the *Bacillus licheniformis* α-amylase gene *(amyL),* the promoters of the *Bacillus stearothermophilus* maltogenic amylase gene *(amyM),* the promoters of the *Bacillus amyloliquefaciens α-*amylase gene *(amyQ),* the promoters of the *Bacillus subtilis xylA and xylB* genes and a promoter derived from a *Lactococcus* sp.-derived promoter including the P170 promoter. When the nucleotide sequence is expressed in a bacterial species such as *E. coli, a* suitable promoter can be selected, for example, from a bacteriophage promoter including a T7 promoter and a phage lambda promoter.

For transcription in a fungal species, examples of useful promoters are those derived from the genes encoding the, *Aspergillus oryzae* TAKA amylase, *Rhizomucor miehei* aspartic proteinase, *Aspergillus niger* neutral α-amylase, *A. niger* acid stable α-amylase, *A. niger glucoamylase, Rhizomucor miehei lipase, Aspergillus oryzae* alkaline protease, *Aspergillus oryzae* triose phosphate isomerase or *Aspergillus nidulans* acetamidase.

Examples of strong constitutive and/or inducible promoters which are preferred for use in fungal expression hosts are those which are obtainable from the fungal genes for xylanase *(xlnA),* phytase, ATP-synthetase, subunit 9 (*oli*C), triose phosphate isomerase (*tpi*), alcohol dehydrogenase *(Adh*A*),* α-amylase (*amy*), amyloglucosidase (AG - from the *gla*A gene), acetamidase (*amd*S) and glyceraldehyde-3-phosphate dehydrogenase (*gpd*) promoters. Other examples of useful promoters for transcription in a fungal host are those derived from the gene encoding *A. oryzae* TAKA amylase, the TPI (triose phosphate isomerase) promoter from S. *cerevisiae* (Alber et al (1982) J. Mol. Appl. Genet. 1, p419-434), *Rhizomucor miehei* aspartic proteinase, *A. niger* neutral α-amylase, *A. niger* acid stable α-amylase, *A. niger* glucoamylase, *Rhizomucor miehei* lipase, *A. oryzae* alkaline protease, *A oryzae* triose phosphate isomerase or *A. nidulans* acetamidase.

Examples of suitable promoters for the expression in a yeast species include but are not limited to the Gal 1 and Gal 10 promoters of *Saccharomyces cerevisiae* and the *Pichia pastoris AOX1 or* AOX2 promoters.

Hybrid promoters may also be used to improve inducible regulation of the expression construct.

The promoter can additionally include features to ensure or to increase expression in a suitable host. For example, the features can be conserved regions such as a Pribnow Box or a TATA box. The promoter may even contain other sequences to affect (such as to maintain, enhance, decrease) the levels of expression of the nucleotide sequence of the present invention. For example, suitable other sequences include the Shl-intron or an ADH intron. Other sequences include inducible elements - such as temperature, chemical, light or stress inducible elements. Also, suitable elements to enhance transcription or translation may be present. An example of the latter element is the TMV 5' signal sequence (see Sleat 1987 Gene 217, 217-225 and Dawson 1993 Plant Mol. Biol. 23: 97).

### CONSTRUCTS

The term "construct" - which is synonymous with terms such as "conjugate", "cassette" and "hybrid" - includes a nucleotide sequence for use according to the present invention directly or indirectly attached to a promoter. An example of an indirect attachment is the provision of a suitable spacer group such as an intron sequence, such as the Sh1-intron or the ADH intron, intermediate the promoter and the nucleotide sequence of the present invention. The same is true for the term "fused" in relation to the present invention which includes direct or indirect attachment. In some cases, the terms do not cover the natural combination of the nucleotide sequence coding for the protein ordinarily associated with the wild type gene promoter and when they are both in their natural environment.

The construct may even contain or express a marker which allows for the selection of the genetic construct in, for example, a bacterium, preferably of the genus Bacillus, such as *Bacillus subtilis,* or plants into which it has been transferred. Various markers exist which may be used, such as for example those encoding mannose-6-phosphate isomerase (especially for plants) or those markers that provide for antibiotic resistance - e.g. resistance to G418, hygromycin, bleomycin, kanamycin and gentamycin.

For some applications, preferably the construct of the present invention comprises at least the nucleotide sequence of the present invention operably linked to a promoter.

### HOST CELLS

The term "host cell" - in relation to the present invention includes any cell that comprises either the nucleotide sequence or an expression vector as described above and which is used in the recombinant production of an enzyme having the specific properties as defined herein. The nucleotide of interest may be homologous or heterologous to the host cell.

Thus, a further embodiment of the present invention provides host cells transformed or transfected with a nucleotide sequence that expresses the enzyme of the present invention. Preferably said nucleotide sequence is carried in a vector for the replication and expression of the nucleotide sequence. The cells will be chosen to be compatible with the said vector and may for example be prokaryotic (for example bacterial), fungal, yeast or plant cells.

Examples of suitable bacterial host organisms are gram positive bacterial species such *as Bacillaceae including Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus lautus, Bacillus megaterium and Bacillus thuringiensis, Streptomyces* species such as *Streptomyces murinus,* lactic acid bacterial species in*cluding Lactococcus* spp. such as *Lactococcus lactis, Lactobacillus* spp. including *Lactobacillus reuteri, Leuconostoc spp., Pediococcus* spp. and *Streptococcus spp.* Alternatively, strains of a gram-negative bacterial species belonging to *Enterobacteriaceae* including *E. coli,* or to *Pseudomonadaceae* can be selected as the host organism.

The gram negative bacterium *E. coli* is widely used as a host for heterologous gene expression. However, large amounts of heterologous protein tend to accumulate inside the cell. Subsequent purification of the desired protein from the bulk of *E. coli* intracellular proteins can sometimes be difficult.

In contrast to *E. coli,* Gram positive bacteria from the genus Bacillus, such as *B. subtilis, B. licheniformis, B. lentus, B. brevis, B. stearothermophilus, B. alkalophilus, B. amyloliquefaciens, B. coagulans, B. circulans, B. lautus, B. megaterium, B. thuringiensis, Streptomyces lividans* or S. *murinus,* may be very suitable as heterologous hosts because of their capability to secrete proteins into the culture medium. Other bacteria that may be suitable as hosts are those from the genera Streptomyces and Pseudomonas.

Depending on the nature of the nucleotide sequence encoding the enzyme of the present invention, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

Typical fungal expression hosts may be selected from *Aspergillus niger, Aspergillus niger var. tubigenis, Aspergillus niger var. awamori, Aspergillus aculeatis, Aspergillus nidulans, Aspergillus oryzae, Trichoderma reesei, Bacillus subtilis, Bacillus licheniformis, Bacillus amyloliquefaciens, Kluyveromyces lactis* and *Saccharomyces cerevisiae.*

Suitable filamentous fungus may be for example a strain belonging to a species of Aspergillus, such as *Aspergillus oryzae* or *Aspergillus niger,* or a strain of *Fusarium oxysporium, Fusarium graminearum* (in the perfect state named *Gribberella zeae,* previously *Sphaeria zeae,* synonym with *Gibberella roseum* and *Gibberella roseum* f. sp. *Cerealis),* or *Fusarium sulphureum* (in the perfect state named *Gibberella puricaris,* synonym with *Fusarium trichothercioides, Fusarium bactridioides, Fusarium sambucium, Fusarium roseum* and *Fusarium roseum var. graminearum), Fusarium cerealis* (synonym with *Fusarium crokkwellnse)* or *Fusarium venenatum.*

Suitable yeast organisms may be selected from the species of *Kluyveromyces, Saccharomyces or Schizosaccharomyces,* e.g. *Saccharomyces cerevisiae,* or *Hansenula* (disclosed in UK Patent Application No. 9927801.2).

The use of suitable host cells - such as yeast, fungal and plant host cells - may provide for post-translational modifications (e.g. myristoylation, glycosylation, truncation, lapidation and tyrosine, serine or threonine phosphorylation) as may be needed to confer optimal biological activity on recombinant expression products of the present invention.

The host cell may be a protease deficient or protease minus strain. This may for example be the protease deficient strain *Aspergillus oryzae* JaL 125 having the alkaline protease gene named "alp" deleted. This strain is described in WO97/35956.

### ORGANISM

The term "organism" in relation to the present invention includes any organism that could comprise the nucleotide sequence coding for the enzyme according to the present invention and/or products obtained therefrom, and/or wherein a promoter can allow expression of the nucleotide sequence according to the present invention when present in the organism.

Suitable organisms may include a prokaryote, fungus, yeast or a plant.

The term "transgenic organism" in relation to the present invention includes any organism that comprises the nucleotide sequence coding for the enzyme according to the present invention and/or the products obtained therefrom, and/or wherein a promoter can allow expression of the nucleotide sequence according to the present invention within the organism. Preferably the nucleotide sequence is incorporated in the genome of the organism.

The term "transgenic organism" does not cover native nucleotide coding sequences in their natural environment when they are under the control of their native promoter which is also in its natural environment.

Therefore, the transgenic organism of the present invention includes an organism comprising any one of, or combinations of, the nucleotide sequence coding for the enzyme according to the present invention, constructs according to the present invention, vectors according to the present invention, plasmids according to the present invention, cells according to the present invention, tissues according to the present invention, or the products thereof. For example the transgenic organism can also comprise the nucleotide sequence coding for the enzyme of the present invention under the control of a heterologous promoter.

### TRANSFORMATION OF HOST CELLS/ORGANISM

As indicated earlier, the host organism can be a prokaryotic or a eukaryotic organism. Examples of suitable prokaryotic hosts include *E*. *coli* and *Bacillus subtilis.*

Teachings on the transformation of prokaryotic hosts is well documented in the art, for example see Sambrook et al (Molecular Cloning: A Laboratory Manual, 2nd edition, 1989, Cold Spring Harbor Laboratory Press) and Ausubel et al., Current Protocols in Molecular Biology (1995), John Wiley & Sons, Inc. If a prokaryotic host is used then the nucleotide sequence may need to be suitably modified before transformation - such as by removal of introns.

Filamentous fungi cells may be transformed by a process involving protoplast formation and transformation of the protoplasts followed by regeneration of the cell wall in a manner known. The use of *Aspergillus* as a host microorganism is described in EP 0 238 023.

Another host organism can be a plant. The basic principle in the construction of genetically modified plants is to insert genetic information in the plant genome so as to obtain a stable maintenance of the inserted genetic material. Several techniques exist for inserting the genetic information, the two main principles being direct introduction of the genetic information and introduction of the genetic information by use of a vector system. A review of the general techniques may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

General teachings on the transformation of fungi, yeasts and plants are presented in following sections.

### TRANSFORMED FUNGUS

A host organism may be a fungus - such as a mold. Examples of suitable such hosts include any member belonging to the genera Phanerochaete, Thermomyces, Acremonium, Aspergillus, Penicillium, Mucor, Neurospora, Trichoderma and the like - such as *Thermomyces lanuginosis, Acremonium chrysogenum, Aspergillus niger, Aspergillus oryzae, Aspergillus awamori, Penicillinum chrysogenem, Mucor javanious, Neurospora crassa, Trichoderma viridae, Phanerochaete chrysosporium,* and the like.

In one embodiment, the host organism may be a filamentous fungus.

For almost a century, filamentous fungi have been widely used in many types of industry for the production of organic compounds and enzymes. For example, traditional Japanese koji and soy fermentations have used *Aspergillus sp.* Also, in this century *Aspergillus niger* has been used for production of organic acids particular citric acid and for production of various enzymes for use in industry.

There are two major reasons why filamentous fungi have been so widely used in industry. First filamentous fungi can produce high amounts of extracellular products, for example enzymes and organic compounds such as antibiotics or organic acids. Second filamentous fungi can grow on low cost substrates such as grains, bran, beet pulp etc. The same reasons have made filamentous fungi attractive organisms as hosts for heterologous expression according to the present invention.

In order to prepare the transgenic *Aspergillus,* expression constructs are prepared by inserting the nucleotide sequence according to the present invention into a construct designed for expression in filamentous fungi.

Several types of constructs used for heterologous expression have been developed. These constructs preferably contain one or more of: a signal sequence which directs the amino acid sequence to be secreted, typically being of fungal origin, and a terminator (typically being active in fungi) which ends the expression system.

Another type of expression system has been developed in fungi where the nucleotide sequence according to the present invention can be fused to a smaller or a larger part of a fungal gene encoding a stable protein. This can stabilise the amino acid sequence. In such a system a cleavage site, recognised by a specific protease, can be introduced between the fungal protein and the amino acid sequence, so the produced fusion protein can be cleaved at this position by the specific protease thus liberating the amino acid sequence. By way of example, one can introduce a site which is recognised by a KEX-2 like peptidase found in at least some *Aspergilli.* Such a fusion leads to cleavage *in vivo* resulting in production of the expressed product and not a larger fusion protein.

Heterologous expression in *Aspergillus* has been reported for several genes coding for bacterial, fungal, vertebrate and plant proteins. The proteins can be deposited intracellularly if the nucleotide sequence according to the present invention is not fused to a signal sequence. Such proteins will accumulate in the cytoplasm and will usually not be glycosylated which can be an advantage for some bacterial proteins. If the nucleotide sequence according to the present invention is equipped with a signal sequence the protein will accumulate extracellularly.

With regard to product stability and host strain modifications, some heterologous proteins are not very stable when they are secreted into the culture fluid of fungi. Most fungi produce several extracellular proteases which degrade heterologous proteins. To avoid this problem special fungal strains with reduced protease production have been used as host for heterologous production.

Teachings on transforming filamentous fungi are reviewed in US-A-5741665 which states that standard techniques for transformation of filamentous fungi and culturing the fungi are well known in the art. An extensive review of techniques as applied to *N*. *crassa* is found, for example in Davis and de Serres, Methods Enzymol (1971) 17A:79-143. Standard procedures are generally used for the maintenance of strains and the preparation of conidia. Mycelia are typically grown in liquid cultures for about 14 hours (25°C), as described in Lambowitz et al., J Cell Biol (1979) 82:17-31. Host strains can generally be grown in either Vogel's or Fries minimal medium supplemented with the appropriate nutrient(s), such as, for example, any one or more of: his, arg, phe, tyr, trp, p-aminobenzoic acid, and inositol.

Further teachings on transforming filamentous fungi are reviewed in US-A-5674707 which states that once a construct has been obtained, it can be introduced either in linear form or in plasmid form, e.g., in a pUC-based or other vector, into a selected filamentous fungal host using a technique such as DNA-mediated transformation, electroporation, particle gun bombardment, protoplast fusion and the like. In addition, Ballance 1991 *(ibid)* states that transformation protocols for preparing transformed fungi are based on preparation of protoplasts and introduction of DNA into the protoplasts using PEG and Ca²⁺ ions. The transformed protoplasts then regenerate and the transformed fungi are selected using various selective markers.

To allow for selection of the resulting transformants, the transformation typically also involves a selectable gene marker which is introduced with the expression cassette, either on the same vector or by co-transformation, into a host strain in which the gene marker is selectable. Various marker/host systems are available, including the pyrG, argB and niaD genes for use with auxotrophic strains of Aspergillus nidulans; pyrG and argB genes for Aspergillus oryzae auxotrophs; pyrG, trpC and niaD genes for *Penicillium chrysogenum* auxotrophs; and the argB gene for *Trichoderma reesei* auxotrophs. Dominant selectable markers including amdS, oliC, hyg and phleo are also now available for use with such filamentous fungi as *A. niger, A. oryzae, A. ficuum, P. chrysogenum, Cephalosporium acremonium, Cochliobolus heterostrophus, Glomerella cingulata, Fulvia fulva* and *Leptosphaeria maculans* (for a review see Ward in Modern Microbial Genetics, 1991, Wiley-Liss, Inc., at pages 455-495). A commonly used transformation marker is the *amdS* gene of *A. nidulans* which in high copy number allows the fungus to grow with acrylamide as the sole nitrogen source.

For the transformation of filamentous fungi, several transformation protocols have been developed for many filamentous. Among the markers used for transformation are a number of auxotrophic markers such as *argB, trpC, niaD* and *pyrG,* antibiotic resistance markers such as benomyl resistance, hygromycin resistance and phleomycin resistance.

In one aspect, the host organism can be of the genus *Aspergillus,* such as *Aspergillus niger.*

A transgenic *Aspergillus* according to the present invention can also be prepared by following the teachings of Rambosek, J. and Leach, J. 1987 (Recombinant DNA in filamentous fungi: Progress and Prospects. CRC Crit. Rev. Biotechnol. 6:357-393), Davis R.W. 1994 (Heterologous gene expression and protein secretion in Aspergillus. In: Martinelli S.D., Kinghorn J.R.(Editors) Aspergillus: 50 years on. Progress in industrial microbiology vol 29. Elsevier Amsterdam 1994. pp 525-560), Ballance, D.J. 1991 (Transformation systems for Filamentous Fungi and an Overview of Fungal Gene structure. In: Leong, S.A., Berka R.M. (Editors) Molecular Industrial Mycology. Systems and Applications for Filamentous Fungi. Marcel Dekker Inc. New York 1991. pp 1-29) and Turner G. 1994 (Vectors for genetic manipulation. In: Martinelli S.D., Kinghorn J.R.( Editors) Aspergillus: 50 years on. Progress in industrial microbiology vol 29. Elsevier Amsterdam 1994. pp. 641-666).

### TRANSFORMED YEAST

In another embodiment the transgenic organism can be a yeast.

In this regard, yeast have also been widely used as a vehicle for heterologous gene expression.

By way of example, the species *Saccharomyces cerevisiae* has a long history of industrial use, including its use for heterologous gene expression. Expression of heterologous genes in Saccharomyces cerevisiae has been reviewed by Goodey et al (1987, Yeast Biotechnology, D R Berry et al, eds, pp 401-429, Allen and Unwin, London) and by King et al (1989, Molecular and Cell Biology of Yeasts, E F Walton and G T Yarronton, eds, pp 107-133, Blackie, Glasgow).

For several reasons *Saccharomyces cerevisiae* is well suited for heterologous gene expression. First, it is non-pathogenic to humans and it is incapable of producing certain endotoxins. Second, it has a long history of safe use following centuries of commercial exploitation for various purposes. This has led to wide public acceptability. Third, the extensive commercial use and research devoted to the organism has resulted in a wealth of knowledge about the genetics and physiology as well as large-scale fermentation characteristics of *Saccharomyces cerevisiae.*

A review of the principles of heterologous gene expression in Saccharomyces cerevisiae and secretion of gene products is given by E Hinchcliffe E Kenny (1993, "Yeast as a vehicle for the expression of heterologous genes", Yeasts, Vol 5, Anthony H Rose and J Stuart Harrison, eds, 2nd edition, Academic Press Ltd.).

Several types of yeast vectors are available, including integrative vectors, which require recombination with the host genome for their maintenance, and autonomously replicating plasmid vectors.

In order to prepare the transgenic Saccharomyces, expression constructs are prepared by inserting the nucleotide sequence of the present invention into a construct designed for expression in yeast. Several types of constructs used for heterologous expression have been developed. The constructs may contain a promoter active in yeast, such as a promoter of yeast origin, such as the GAL1 promoter, is used. Usually a signal sequence of yeast origin, such as the sequence encoding the SUC2 signal peptide, is used. A terminator active in yeast ends the expression system.

For the transformation of yeast several transformation protocols have been developed. For example, a transgenic Saccharomyces according to the present invention can be prepared by following the teachings of Hinnen et al (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells may be selected using various selective markers. Among the markers used for transformation are a number of auxotrophic markers such as LEU2, HIS4 and TRP1, and dominant antibiotic resistance markers such as aminoglycoside antibiotic markers, eg G418.

### TRANSFORMED PLANTS/PLANT CELLS

A preferred host organism suitable for the present invention is a plant.

In this respect, the basic principle in the construction of genetically modified plants is to insert genetic information in the plant genome so as to obtain a stable maintenance of the inserted genetic material.

Several techniques exist for inserting the genetic information, the two main principles being direct introduction of the genetic information and introduction of the genetic information by use of a vector system. A review of the general techniques may be found in articles by Potrykus (Annu Rev Plant Physiol Plant Mol Biol [1991] 42:205-225) and Christou (Agro-Food-Industry Hi-Tech March/April 1994 17-27).

Even though the promoter of the present invention is not disclosed in EP-B-0470145 and CA-A-2006454, those two documents do provide some useful background commentary on the types of techniques that may be employed to prepare transgenic plants according to the present invention. Some of these background teachings are now included in the following commentary.

The basic principle in the construction of genetically modified plants is to insert genetic information in the plant genome so as to obtain a stable maintenance of the inserted genetic material.

Thus, in one aspect, the present invention relates to a vector system which carries a nucleotide sequence or construct according to the present invention and which is capable of introducing the nucleotide sequence or construct into the genome of an organism, such as a plant.

The vector system may comprise one vector, but it can comprise two vectors. In the case of two vectors, the vector system is normally referred to as a binary vector system. Binary vector systems are described in further detail in Gynheung An et al. (1980), Binary Vectors, Plant Molecular Biology Manual A3, 1-19.

One extensively employed system for transformation of plant cells with a given promoter or nucleotide sequence or construct is based on the use of a Ti plasmid from *Agrobacterium tumefaciens* or a Ri plasmid from *Agrobacterium rhizogenes* An et al. (1986), Plant Physiol. 81, 301-305 and Butcher D.N. et al. (1980), Tissue Culture Methods for Plant Pathologists, eds.: D.S. Ingrams and J.P. Helgeson, 203-208.

Several different Ti and Ri plasmids have been constructed which are suitable for the construction of the plant or plant cell constructs described above. A non-limiting example of such a Ti plasmid is pGV3850.

The nucleotide sequence or construct of the present invention should preferably be inserted into the Ti-plasmid between the terminal sequences of the T-DNA or adjacent a T-DNA sequence so as to avoid disruption of the sequences immediately surrounding the T-DNA borders, as at least one of these regions appear to be essential for insertion of modified T-DNA into the plant genome.

As will be understood from the above explanation, if the organism is a plant, then the vector system of the present invention is preferably one which contains the sequences necessary to infect the plant (e.g. the *vir* region) and at least one border part of a T-DNA sequence, the border part being located on the same vector as the genetic construct. Preferably, the vector system is an *Agrobacterium tumefaciens* Ti-plasmid or an *Agrobacterium rhizogenes* Ri-plasmid or a derivative thereof, as these plasmids are well-known and widely employed in the construction of transgenic plants, many vector systems exist which are based on these plasmids or derivatives thereof.

In the construction of a transgenic plant the nucleotide sequence or construct of the present invention may be first constructed in a micro-organism in which the vector can replicate and which is easy to manipulate before insertion into the plant. An example of a useful micro-organism is *E. coli.,* but other micro-organisms having the above properties may be used. When a vector of a vector system as defined above has been constructed in *E. coli.* it is transferred, if necessary, into a suitable *Agrobacterium* strain, e.g. *Agrobacterium tumefaciens.* The Ti-plasmid harbouring the nucleotide sequence or construct of the invention is thus preferably transferred into a suitable *Agrobacterium* strain, e.g. *A. tumefaciens,* so as to obtain an *Agrobacterium* cell harbouring the nucleotide sequence or construct of the invention, which DNA is subsequently transferred into the plant cell to be modified.

As reported in CA-A-2006454, a large amount of cloning vectors are available which contain a replication system in *E*. *coli* and a marker which allows a selection of the transformed cells. The vectors contain for example pBR 322, the pUC series, the M13 mp series, pACYC 184 etc.

In this way, the nucleotide or construct of the present invention can be introduced into a suitable restriction position in the vector. The contained plasmid is used for the transformation in *E.coli.* The *E.coli* cells are cultivated in a suitable nutrient medium and then harvested and lysed. The plasmid is then recovered. As a method of analysis there is generally used sequence analysis, restriction analysis, electrophoresis and further biochemical-molecular biological methods. After each manipulation, the used DNA sequence can be restricted and connected with the next DNA sequence. Each sequence can be cloned in the same or different plasmid.

After each introduction method of the desired promoter or construct or nucleotide sequence according to the present invention in the plants the presence and/or insertion of further DNA sequences may be necessary. If, for example, for the transformation the Ti- or Ri-plasmid of the plant cells is used, at least the right boundary and often however the right and the left boundary of the Ti- and Ri-plasmid T-DNA, as flanking areas of the introduced genes, can be connected. The use of T-DNA for the transformation of plant cells has been intensively studied and is described in EP-A-120516; Hoekema, in: The Binary Plant Vector System Offset-drukkerij Kanters B.B., Alblasserdam, 1985, Chapter V; Fraley, et al., Crit. Rev. Plant Sci., 4:1-46; and An et al., EMBO J. (1985) 4:277-284.

Direct infection of plant tissues by *Agrobacterium* is a simple technique which has been widely employed and which is described in Butcher D.N. et al. (1980), Tissue Culture Methods for Plant Pathologists, eds.: D.S. Ingrams and J.P. Helgeson, 203-208. For further teachings on this topic see Potrykus (Annu Rev Plant Physiol Plant Mol Biol [1991] 42:205-225) and Christou (Agro-Food-Industry Hi-Tech March/April 1994 17-27). With this technique, infection of a plant may be done on a certain part or tissue of the plant, i.e. on a part of a leaf, a root, a stem or another part of the plant.

Typically, with direct infection of plant tissues by *Agrobacterium* carrying the promoter and/or the GOI, a plant to be infected is wounded, e.g. by cutting the plant with a razor or puncturing the plant with a needle or rubbing the plant with an abrasive. The wound is then inoculated with the *Agrobacterium.* The inoculated plant or plant part is then grown on a suitable culture medium and allowed to develop into mature plants.

When plant cells are constructed, these cells may be grown and maintained in accordance with well-known tissue culturing methods such as by culturing the cells in a suitable culture medium supplied with the necessary growth factors such as amino acids, plant hormones, vitamins, etc. Regeneration of the transformed cells into genetically modified plants may be accomplished using known methods for the regeneration of plants from cell or tissue cultures, for example by selecting transformed shoots using an antibiotic and by subculturing the shoots on a medium containing the appropriate nutrients, plant hormones, etc.

Other techniques for transforming plants include ballistic transformation, the silicon whisker carbide technique (see Frame BR, Drayton PR, Bagnaall SV, Lewnau CJ, Bullock WP, Wilson HM, Dunwell JM, Thompson JA & Wang K (1994) Production of fertile transgenic maize plants by silicon carbide whisker-mediated transformation, The Plant Journal 6: 941-948) and viral transformation techniques (e.g. see Meyer P, Heidmann I & Niedenhof I (1992) The use of cassava mosaic virus as a vector system for plants, Gene 110: 213-217). Teachings on ballistic transformation are presented in following section.

Further teachings on plant transformation may be found in EP-A-0449375.

### BALLISTIC TRANSFORMATION OF PLANTS AND PLANT TISSUE

As indicated, techniques for producing transgenic plants are well known in the art. Typically, either whole plants, cells or protoplasts may be transformed with a suitable nucleic acid construct encoding a zinc finger molecule or target DNA (see above for examples of nucleic acid constructs). There are many methods for introducing transforming DNA constructs into cells, but not all are suitable for delivering DNA to plant cells. Suitable methods include *Agrobacterium* infection (see, among others, Turpen et al., 1993, J. Virol. Methods, 42: 227-239) or direct delivery of DNA such as, for example, by PEG-mediated transformation, by electroporation or by acceleration of DNA coated particles. Acceleration methods are generally preferred and include, for example, microprojectile bombardment.

Originally developed to produce stable transformants of plant species which were recalcitrant to transformation by *Agrobacterium tumefaciens,* ballistic transformation of plant tissue, which introduces DNA into cells on the surface of metal particles, has found utility in testing the performance of genetic constructs during transient expression. In this way, gene expression can be studied in transiently transformed cells, without stable integration of the gene in interest, and thereby without time-consuming generation of stable transformants.

In more detail, the ballistic transformation technique (otherwise known as the particle bombardment technique) was first described by Klein *et al.* [1987], Sanford *et al.* **[1987]** and Klein *et al.* [1988] and has become widespread due to easy handling and the lack of pre-treatment of the cells or tissue in interest.

The principle of the particle bombardment technique is direct delivery of DNA-coated micro-projectiles into intact plant cells by a driving force (e.g. electrical discharge or compressed air). The micro-projectiles penetrate the cell wall and membrane, with only minor damage, and the transformed cells then express the promoter constructs.

One particle bombardment technique that can be performed uses the Particle Inflow Gun (PIG), which was developed and described by Finer *et al.* [1992] and Vain *et al.* [1993]. The PIG accelerates the micro-projectiles in a stream of flowing helium, through a partial vacuum, into the plant cells.

One of advantages of the PIG is that the acceleration of the micro-projectiles can be controlled by a timer-relay solenoid and by regulation the provided helium pressure. The use of pressurised helium as a driving force has the advantage of being inert, leaves no residues and gives reproducible acceleration. The vacuum reduces the drag on the particles and lessens tissue damage by dispersion of the helium gas prior to impact [Finer *et al.* 1992].

In some cases, the effectiveness and ease of the PIG system makes it a good choice for the generation of transient transformed guar tissue, which were tested for transient expression of promoter/reporter gene fusions.

A typical protocol for producing transgenic plants (in particular moncotyledons), taken from U.S. Patent No. 5, 874, 265, is described below.

An example of a method for delivering transforming DNA segments to plant cells is microprojectile bombardment. In this method, non-biological particles may be coated with nucleic acids and delivered into cells by a propelling force. Exemplary particles include those comprised of tungsten, gold, platinum, and the like.

A particular advantage of microprojectile bombardment, in addition to it being an effective means of reproducibly stably transforming both dicotyledons and monocotyledons, is that neither the isolation of protoplasts nor the susceptibility to *Agrobacterium* infection is required. An illustrative embodiment of a method for delivering DNA into plant cells by acceleration is a Biolistics Particle Delivery System, which can be used to propel particles coated with DNA through a screen, such as a stainless steel or Nytex screen, onto a filter surface covered with plant cells cultured in suspension. The screen disperses the tungsten-DNA particles so that they are not delivered to the recipient cells in large aggregates. It is believed that without a screen intervening between the projectile apparatus and the cells to be bombarded, the projectiles aggregate and may be too large for attaining a high frequency of transformation. This may be due to damage inflicted on the recipient cells by projectiles that are too large.

For the bombardment, cells in suspension are preferably concentrated on filters. Filters containing the cells to be bombarded are positioned at an appropriate distance below the macroprojectile stopping plate. If desired, one or more screens are also positioned between the gun and the cells to be bombarded. Through the use of techniques set forth herein one may obtain up to 1000 or more clusters of cells transiently expressing a marker gene ("foci") on the bombarded filter. The number of cells in a focus which express the exogenous gene product 48 hours post-bombardment often range from 1 to 10 and average 2 to 3.

After effecting delivery of exogenous DNA to recipient cells by any of the methods discussed above, a preferred step is to identify the transformed cells for further culturing and plant regeneration. This step may include assaying cultures directly for a screenable trait or by exposing the bombarded cultures to a selective agent or agents.

An example of a screenable marker trait is the red pigment produced under the control of the R-locus in maize. This pigment may be detected by culturing cells on a solid support containing nutrient media capable of supporting growth at this stage, incubating the cells at, e.g., 18°C and greater than 180 µE m⁻² s⁻¹, and selecting cells from colonies (visible aggregates of cells) that are pigmented. These cells may be cultured further, either in suspension or on solid media.

An exemplary embodiment of methods for identifying transformed cells involves exposing the bombarded cultures to a selective agent, such as a metabolic inhibitor, an antibiotic, herbicide or the like. Cells which have been transformed and have stably integrated a marker gene conferring resistance to the selective agent used, will grow and divide in culture. Sensitive cells will not be amenable to further culturing.

To use the bar-bialaphos selective system, bombarded cells on filters are resuspended in nonselective liquid medium, cultured (e.g. for one to two weeks) and transferred to filters overlaying solid medium containing from 1-3 mg/l bialaphos. While ranges of 1-3 mg/l will typically be preferred, it is proposed that ranges of 0.1-50 mg/l will find utility in the practice of the invention. The type of filter for use in bombardment is not believed to be particularly crucial, and can comprise any solid, porous, inert support.

Cells that survive the exposure to the selective agent may be cultured in media that supports regeneration of plants. Tissue is maintained on a basic media with hormones for about 2-4 weeks, then transferred to media with no hormones. After 2-4 weeks, shoot development will signal the time to transfer to another media.

Regeneration typically requires a progression of media whose composition has been modified to provide the appropriate nutrients and hormonal signals during sequential developmental stages from the transformed callus to the more mature plant. Developing plantlets are transferred to soil, and hardened, e.g., in an environmentally controlled chamber at about 85% relative humidity, 600 ppm CO₂, and 250 µE m⁻² s⁻¹ of light. Plants are preferably matured either in a growth chamber or greenhouse. Regeneration will typically take about 3-12 weeks. During regeneration, cells are grown on solid media in tissue culture vessels. An illustrative embodiment of such a vessel is a petri dish. Regenerating plants are preferably grown at about 19°C to 28°C. After the regenerating plants have reached the stage of shoot and root development, they may be transferred to a greenhouse for further growth and testing.

Genomic DNA may be isolated from callus cell lines and plants to determine the presence of the exogenous gene through the use of techniques well known to those skilled in the art such as PCR and/or Southern blotting.

Several techniques exist for inserting the genetic information, the two main principles being direct introduction of the genetic information and introduction of the genetic information by use of a vector system. A review of the general techniques may be found in articles by Potrykus (Annu Rev Plant Physiol Plant Mol Biol [1991] 42:205-225) and Christou (Agro-Food-Industry Hi-Tech March/April 1994 17-27).

### CULTURING AND PRODUCTION

Host cells transformed with the nucleotide sequence may be cultured under conditions conducive to the production of the encoded enzyme and which facilitate recovery of the enzyme from the cells and/or culture medium.

The medium used to cultivate the cells may be any conventional medium suitable for growing the host cell in questions and obtaining expression of the enzyme. Suitable media are available from commercial suppliers or may be prepared according to published recipes (e.g. as described in catalogues of the American Type Culture Collection).

The protein produced by a recombinant cell may be displayed on the surface of the cell. If desired, and as will be understood by those of skill in the art, expression vectors containing coding sequences can be designed with signal sequences which direct secretion of the coding sequences through a particular prokaryotic or eukaryotic cell membrane. Other recombinant constructions may join the coding sequence to nucleotide sequence encoding a polypeptide domain which will facilitate purification of soluble proteins (Kroll DJ et al (1993) DNA Cell Biol 12:441-53).

The enzyme may be secreted from the host cells and may conveniently be recovered from the culture medium by well-known procedures, including separating the cells from the medium by centrifugation or filtration, and precipitating proteinaceous components of the medium by means of a salt such as ammonium sulphate, followed by the use of chromatographic procedures such as ion exchange chromatography, affinity chromatography, or the like.

### SECRETION

Often, it is desirable for the enzyme to be secreted from the expression host into the culture medium from where the enzyme may be more easily recovered. According to the present invention, the secretion leader sequence may be selected on the basis of the desired expression host. Hybrid signal sequences may also be used with the context of the present invention.

Typical examples of heterologous secretion leader sequences are those originating from the fungal amyloglucosidase (AG) gene *(gla*A *-* both 18 and 24 amino acid versions e.g. from *Aspergillus),* the a-factor gene (yeasts e.g. *Saccharomyces, Kluyveromyces* and *Hansenula)* or the α-amylase gene *(Bacillus).*

### DETECTION

A variety of protocols for detecting and measuring the expression of the amino acid sequence are known in the art. Examples include enzyme-linked immunosorbent assay (ELISA), radioimmunoassay (RIA) and fluorescent activated cell sorting (FACS). A two-site, monoclonal-based immunoassay utilizing monoclonal antibodies reactive to two non-interfering epitopes on the POI may be used or a competitive binding assay may be employed. These and other assays are described, among other places, in Hampton R et al (1990, Serological Methods, A Laboratory Manual, APS Press, St Paul MN) and Maddox DE et al (1983, J Exp Med 158:1211).

A wide variety of labels and conjugation techniques are known by those skilled in the art and can be used in various nucleic and amino acid assays. Means for producing labelled hybridization or PCR probes for detecting the amino acid sequence include oligolabelling, nick translation, end-labelling or PCR amplification using a labelled nucleotide. Alternatively, the NOI, or any portion of it, may be cloned into a vector for the production of an mRNA probe. Such vectors are known in the art, are commercially available, and may be used to synthesize RNA probes *in vitro* by addition of an appropriate RNA polymerase such as T7, T3 or SP6 and labeled nucleotides.

A number of companies such as Pharmacia Biotech (Piscataway, NJ), Promega (Madison, WI), and US Biochemical Corp (Cleveland, OH) supply commercial kits and protocols for these procedures. Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3,817,837; US-A-3,850,752; US-A-3,939,350; US-A-3,996,345; US-A-4,277,437; US-A-4,275,149 and US-A-4,366,241. Also, recombinant immunoglobulins may be produced as shown in US-A-4,816,567.

Additional methods to quantitate the expression of the amino acid sequence include radiolabeling (Melby PC et al 1993 J Immunol Methods 159:235-44) or biotinylating (Duplaa C et al 1993 Anal Biochem 229-36) nucleotides, coamplification of a control nucleic acid, and standard curves onto which the experimental results are interpolated. Quantitation of multiple samples may be speeded up by running the assay in an ELISA format where the oligomer of interest is presented in various dilutions and a spectrophotometric or calorimetric response gives rapid quantitation.

Although the presence/absence of marker gene expression suggests that the nucleotide sequence is also present, its presence and expression should be confirmed. For example, if the nucleotide sequence is inserted within a marker gene sequence, recombinant cells containing nucleotide sequences can be identified by the absence of marker gene function. Alternatively, a marker gene can be placed in tandem with a nucleotide sequence under the control of the promoter of the present invention or an alternative promoter (preferably the same promoter of the present invention). Expression of the marker gene in response to induction or selection usually indicates expression of the amino acid sequence as well.

Alternatively, host cells which contain the nucleotide sequence may be identified by a variety of procedures known to those of skill in the art. These procedures include, but are not limited to, DNA-DNA or DNA-RNA hybridization and protein bioassay or immunoassay techniques which include membrane-based, solution-based, or chip-based technologies for the detection and/or quantification of the nucleic acid or protein.

### FUSION PROTEINS

The amino acid sequence of the present invention may be produced as a fusion protein, for example to aid in extraction and purification. Examples of fusion protein partners include glutathione-S-transferase (GST), 6xHis, GAL4 (DNA binding and/or transcriptional activation domains) and (β-galactosidase. It may also be convenient to include a proteolytic cleavage site between the fusion protein partner and the protein sequence of interest to allow removal of fusion protein sequences. Preferably the fusion protein will not hinder the activity of the protein sequence.

The fusion protein may comprise an antigen or an antigenic determinant fused to the substance of the present invention. In this embodiment, the fusion protein may be a non-naturally occurring fusion protein comprising a substance which may act as an adjuvant in the sense of providing a generalised stimulation of the immune system. The antigen or antigenic determinant may be attached to either the amino or carboxy terminus of the substance.

In another embodiment of the invention, the amino acid sequence may be ligated to a heterologous sequence to encode a fusion protein. For example, for screening of peptide libraries for agents capable of affecting the substance activity, it may be useful to encode a chimeric substance expressing a heterologous epitope that is recognised by a commercially available antibody.

### ADDITIONAL POIs

The sequences of the present invention may be used in conjunction with one or more additional proteins of interest (POIs) or nucleotide sequences of interest (NOIs).

Non-limiting examples of POIs include: proteins or enzymes involved in starch metabolism, proteins or enzymes involved in glycogen metabolism, acetyl esterases, aminopeptidases, amylases, arabinases, arabinofuranosidases, carboxypeptidases, catalases, cellulases, chitinases, chymosin, cutinase, deoxyribonucleases, epimerases, esterases, α-galactosidases, β-galactosidases, α-glucanases, glucan lysases, endo-β-glucanases, glucoamylases, glucose oxidases, α-glucosidases, β-glucosidases, glucuronidases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, peroxidases, phenoloxidases, phytases, polygalacturonases, proteases, rhamnogalacturonases, ribonucleases, thaumatin, transferases, transport proteins, transglutaminases, xylanases, hexose oxidase (D-hexose: O₂-oxidoreductase EC 1.1.3.5) or combinations thereof. The NOI may even be an antisense sequence for any of those sequences.

The POI may even be a fusion protein, for example to aid in extraction and purification.

Examples of fusion protein partners include the maltose binding protein, glutathione-S-transferase (GST), 6xHis, GAL4 (DNA binding and/or transcriptional activation domains) and β-galactosidase. It may also be convenient to include a proteolytic cleavage site between the fusion components.

The POI may even be fused to a secretion sequence. Examples of secretion leader sequences are those originating from the amyloglucosidase gene, the α-factor gene, the α-amylase gene, the lipase A gene, the xylanase A gene.

Other sequences can also facilitate secretion or increase the yield of secreted POI. Such sequences could code for chaperone proteins as for example the product of *Aspergillus niger cyp B* gene described in UK patent application 9821198.0.

The NOI may be engineered in order to alter their activity for a number of reasons, including but not limited to, alterations which modify the processing and/or expression of the expression product thereof. For example, mutations may be introduced using techniques which are well known in the art, e.g., site-directed mutagenesis to insert new restriction sites, to alter glycosylation patterns or to change codon preference. By way of further example, the NOI may also be modified to optimise expression in a particular host cell. Other sequence changes may be desired in order to introduce restriction enzyme recognition sites.

The NOI may include within it synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the NOI may be modified by any method available in the art. Such modifications may be carried out in to enhance the *in vivo* activity or life span of the NOI.

The NOI may be modified to increase intracellular stability and half-life. Possible modifications include, but are not limited to, the addition of flanking sequences of the 5' and/or 3' ends of the molecule or the use of phosphorothioate or 2' O-methyl rather than phosphodiesterase linkages within the backbone of the molecule.

### ANTIBODIES

One aspect of the present invention relates to amino acid sequences that are immunologically reactive with one or more of the amino acid sequences of paragraph 1.

Antibodies may be produced by standard techniques, such as by immunisation with the substance of the invention or by using a phage display library.

For the purposes of this invention, the term "antibody", unless specified to the contrary, includes but is not limited to, polyclonal, monoclonal, chimeric, single chain, Fab fragments, fragments produced by a Fab expression library, as well as mimetics thereof. Such fragments include fragments of whole antibodies which retain their binding activity for a target substance, Fv, F(ab') and F(ab')₂ fragments, as well as single chain antibodies (scFv), fusion proteins and other synthetic proteins which comprise the antigen-binding site of the antibody. Furthermore, the antibodies and fragments thereof may be humanised antibodies. Neutralising antibodies, i.e., those which inhibit biological activity of the substance polypeptides, are especially preferred for diagnostics and therapeutics.

If polyclonal antibodies are desired, a selected mammal (e.g., mouse, rabbit, goat, horse, etc.) is immunised with the sequence of the present invention (or a sequence comprising an immunological epitope thereof). Depending on the host species, various adjuvants may be used to increase immunological response. Such adjuvants include, but are not limited to, Freund's, mineral gels such as aluminium hydroxide, and surface active substances such as lysolecithin, pluronic polyols, polyanions, peptides, oil emulsions, keyhole limpet hemocyanin, and dinitrophenol. BCG *(Bacilli Calmette-Guerin)* and *Corynebacterium parvum* are potentially useful human adjuvants which may be employed if purified the substance polypeptide is administered to immunologically compromised individuals for the purpose of stimulating systemic defence.

Serum from the immunised animal is collected and treated according to known procedures. If serum containing polyclonal antibodies to the sequence of the present invention (or a sequence comprising an immunological epitope thereof) contains antibodies to other antigens, the polyclonal antibodies can be purified by immunoaffinity chromatography. Techniques for producing and processing polyclonal antisera are known in the art. In order that such antibodies may be made, the invention also provides polypeptides of the invention or fragments thereof haptenised to another polypeptide for use as immunogens in animals or humans.

Monoclonal antibodies directed against the sequence of the present invention (or a sequence comprising an immunological epitope thereof) can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against orbit epitopes can be screened for various properties; i.e., for isotype and epitope affinity.

Monoclonal antibodies to the sequence of the present invention (or a sequence comprising an immunological epitope thereof) may be prepared using any technique which provides for the production of antibody molecules by continuous cell lines in culture. These include, but are not limited to, the hybridoma technique originally described by Koehler and Milstein (1975 Nature 256:495-497), the human B-cell hybridoma technique (Kosbor et al (1983) Immunol Today 4:72; Cote et al (1983) Proc Natl Acad Sci 80:2026-2030) and the EBV-hybridoma technique (Cole et al (1985) Monoclonal Antibodies and Cancer Therapy, Alan R Liss Inc, pp 77-96). In addition, techniques developed for the production of "chimeric antibodies", the splicing of mouse antibody genes to human antibody genes to obtain a molecule with appropriate antigen specificity and biological activity can be used (Morrison et al (1984) Proc Natl Acad Sci 81:6851-6855; Neuberger et al (1984) Nature 312:604-608; Takeda et al (1985) Nature 314:452-454). Alternatively, techniques described for the production of single chain antibodies (US Patent No. 4,946,779) can be adapted to produce the substance specific single chain antibodies.

Antibodies may also be produced by inducing *in vivo* production in the lymphocyte population or by screening recombinant immunoglobulin libraries or panels of highly specific binding reagents as disclosed in Orlandi et al (1989, Proc Natl Acad Sci 86: 3833-3837), and Winter G and Milstein C (1991; Nature 349:293-299).

Antibody fragments which contain specific binding sites for the substance may also be generated. For example, such fragments include, but are not limited to, the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed to allow rapid and easy identification of monoclonal Fab fragments with the desired specificity (Huse WD et al (1989) Science 256:1275-128 1).

### LARGE SCALE APPLICATION

In one preferred embodiment of the present invention, the amino acid sequence is used for large scale applications.

Preferably the amino acid sequence is produced in a quantity of from 1g per litre to about 2g per litre of the total cell culture volume after cultivation of the host organism.

Preferably the amino acid sequence is produced in a quantity of from 100mg per litre to about 900mg per litre of the total cell culture volume after cultivation of the host organism.

Preferably the amino acid sequence is produced in a quantity of from 250mg per litre to about 500mg per litre of the total cell culture volume after cultivation of the host organism.

### SUMMARY

In summation, the present invention relates to an amino acid sequence and a nucleotide sequence and, also to a construct comprising the same. The invention also relates to new uses of a known enzyme.

The invention is further illustrated in the following non-limiting examples, and with reference to the following figures wherein:

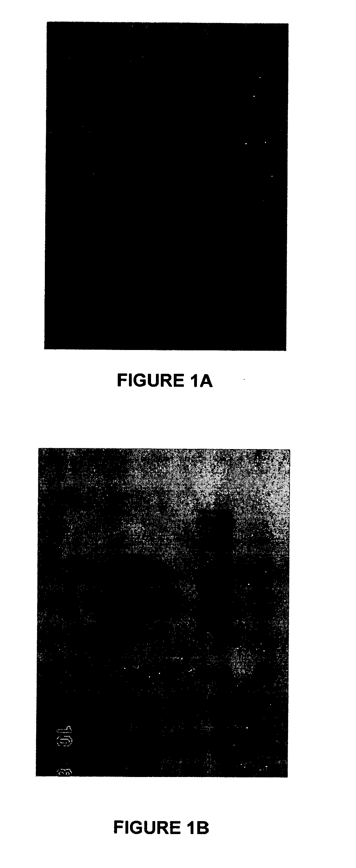

Figure 1 shows the electrophoresis of PD1 (pyranosone dehydratase isoform 1) on gels of 8-25% gradient. In more detail, Figure 1A, shows SDS-PAGE: Lanes 1 and 2 (from left) protein markers from Novex and Pharmacia respectively, Lanes 3, 4 and 5, purified PD1. Figure 1B, shows Native PAGE: Lanes 1, 2, and 3, purified PD 1, Lane 4, protein markers from Pharmacia, Lane 5, partially purified. The gels were stained with PhastGel Blue R from Pharmacia.

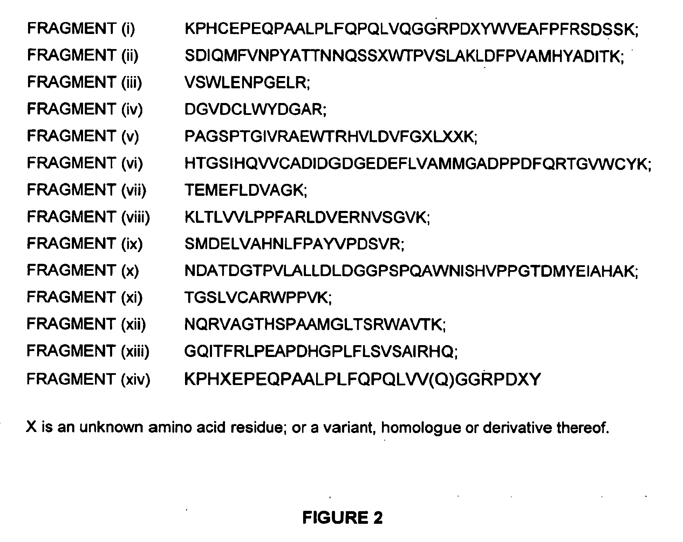

Figure 2 shows partial amino acid sequences of pyranosone dehydratase.

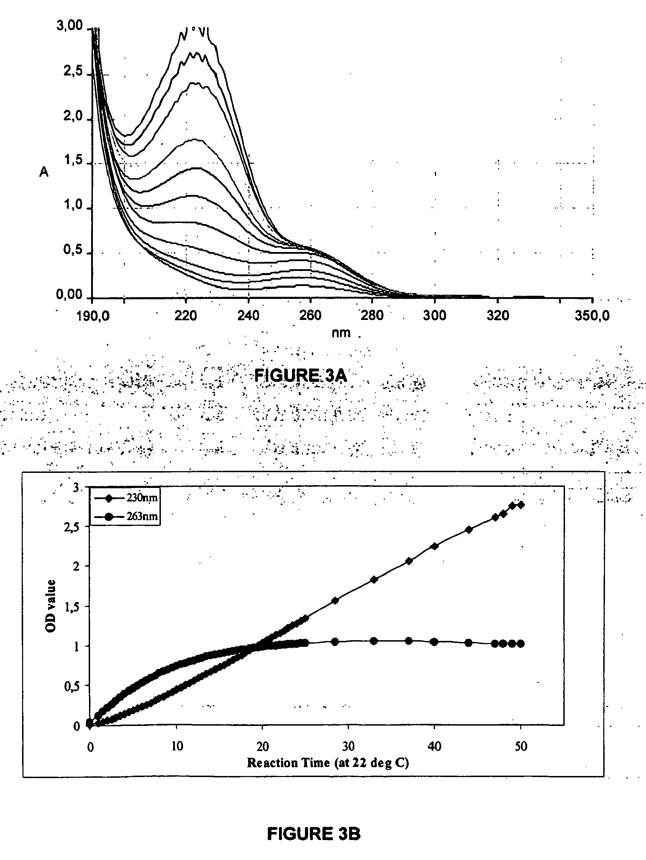

Figure 3A illustrates the use of 1,5-anhydro-D-fructose and PD for the production of microthecin. The reaction mixture consisted of 1,5-Anhydro-D-fructose 5µl (3.0%), PD preparation 5µl, 65µl sodium phosphate buffer (pH 6.0) and water to a final volume of 0.7 ml. The reaction was monitored by scanning between 350-190 nm. Reaction time at zero min was used as blank. The absorbance peak at around 230 nm indicates the formation of microthecin. The absorbance at 265 nm indicate the first formation of an intermediate from AF before it converts to microthecin.
Figure 3B illustrates the production of microthecin and its intermediate. The reaction mixture consisted of 10µl partially purified PD (a ammonium sulfate fraction between 25-50% saturation of the cell-free extract from *Phanerochaete chrysosporium),* 25µl AF (3.0%, w/v), 100µl sodium phosphate buffer (0.1M, pH6.5) and 0.84 ml water. The reaction was started by the addition of the substrate AF. The reaction was performed at 22 °C. The formation of microthecin and its intermediate was monitored at 230 nm and 263 nm, respectively. One can see that the intermediate was first formed and leveled off after around 20 min. There was a delay for the formation of microthecin but its formation continued until nearly all the AF in the reaction mixture was consumed.

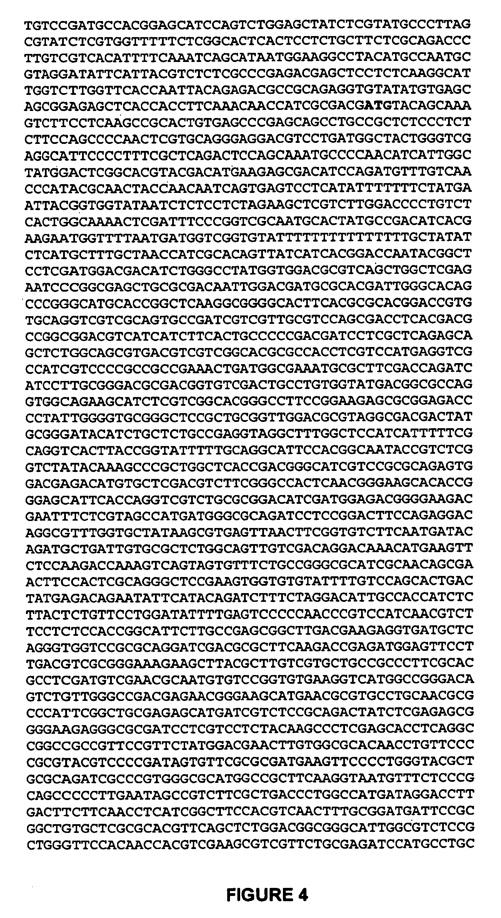

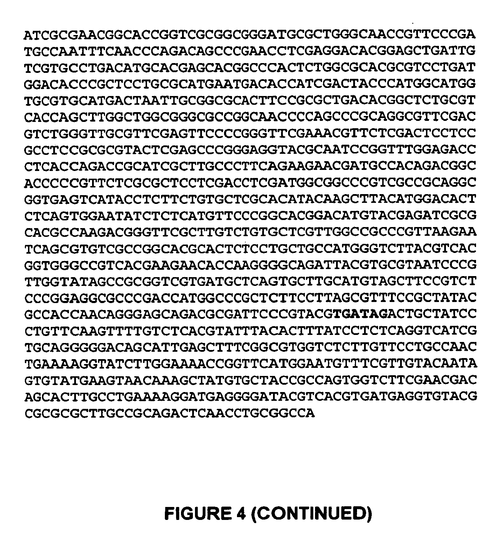

Figure 4 shows SEQ ID NO.1, the gene coding for pyranosone dehydratase (PD) from the fungus *Phanerochaete chrysosporium* including the upstream regulatory region (-1-to -288), the coding region (1-3146) and down-stream region (3147-3444). The presumed starch coden is ATG (bold) and stop codens are TGA TAG(bold). The purified functional PD corresponds to a N-terminal 7-amino acid truncated PD if the translation is supposed to start from the bold coden ATG.

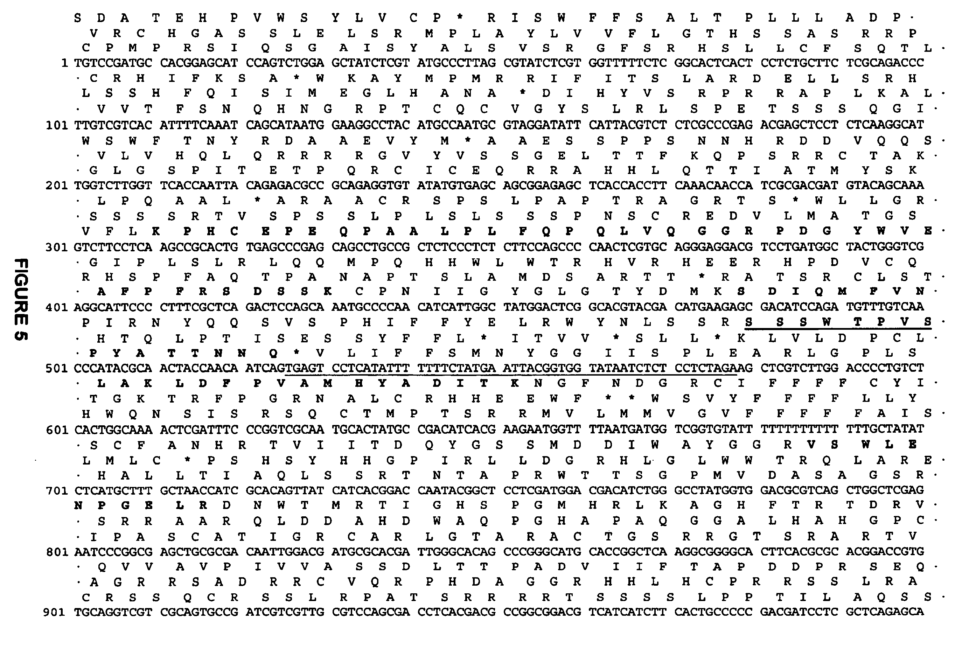

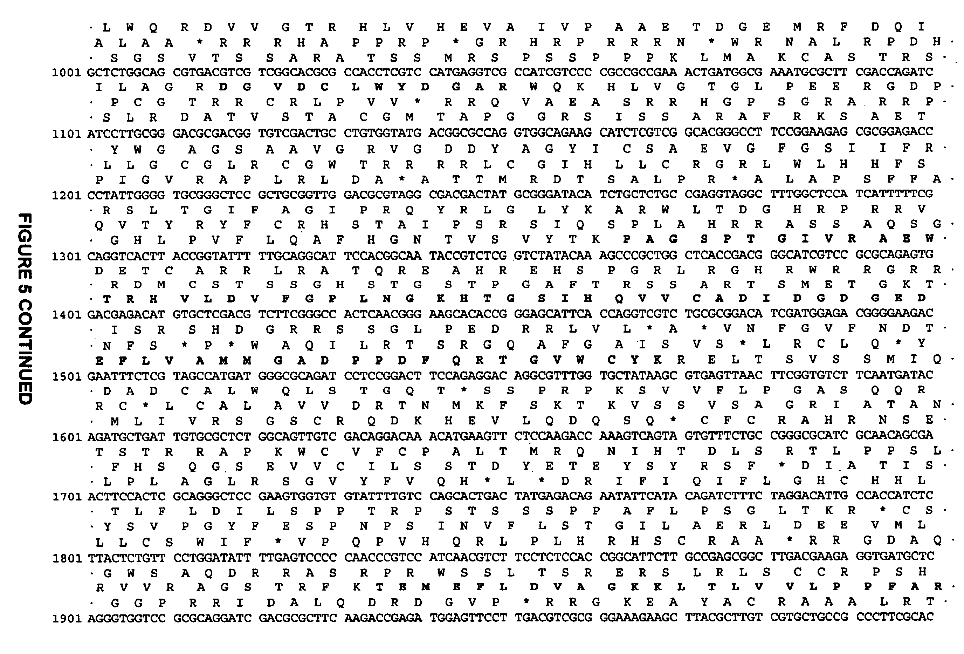

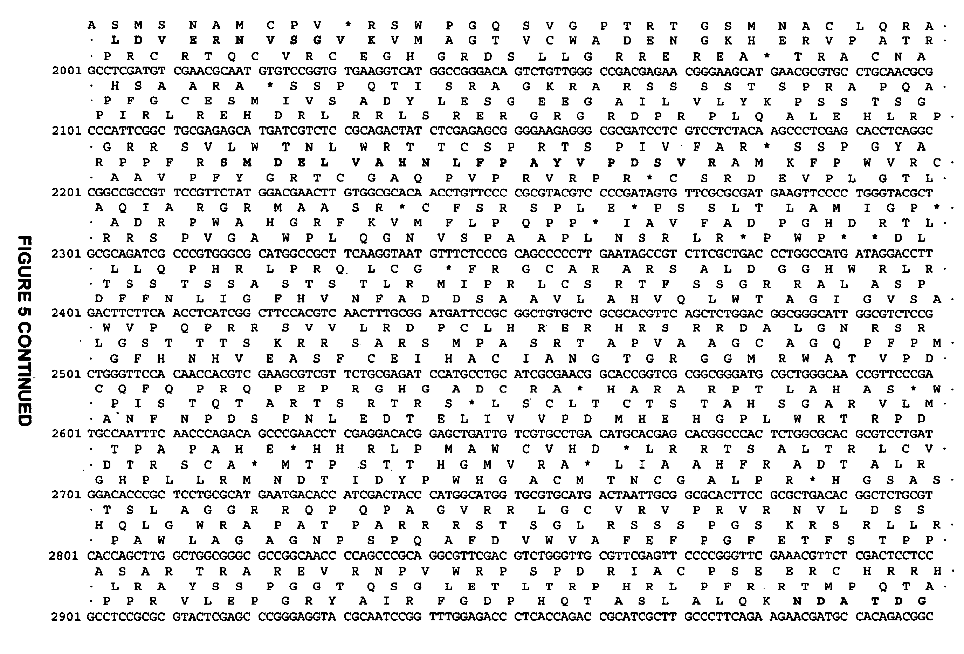

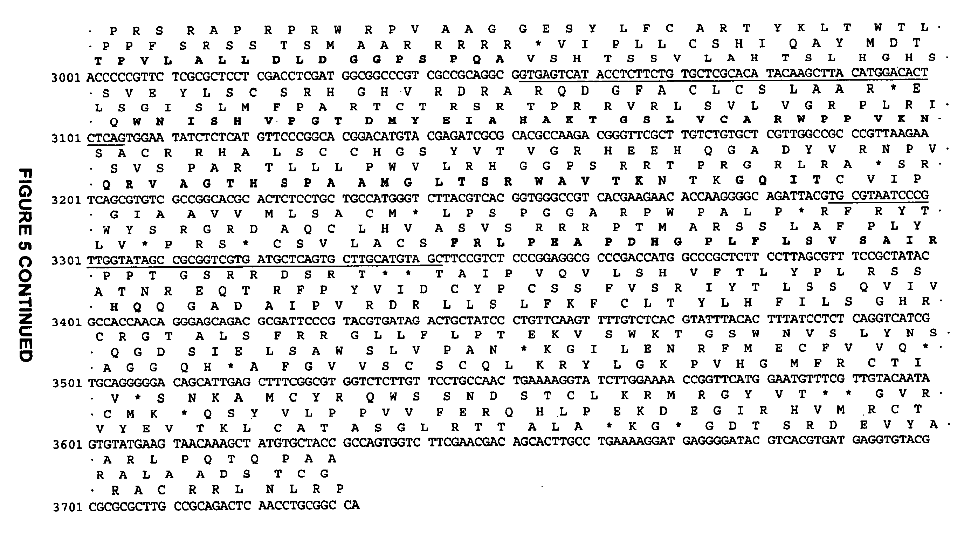

Figure 5 shows the upstream region, the coding region and the down stream region of the pyranosone dehydratase (PD) gene from the fungus *Phanerochaete chrysosporium.* The DNA sequence theoretically could code for three proteins with different amino acid sequences. The bold amino acids are those found by amino acid sequencing of the purified functional PD. Identified introns are underlined.

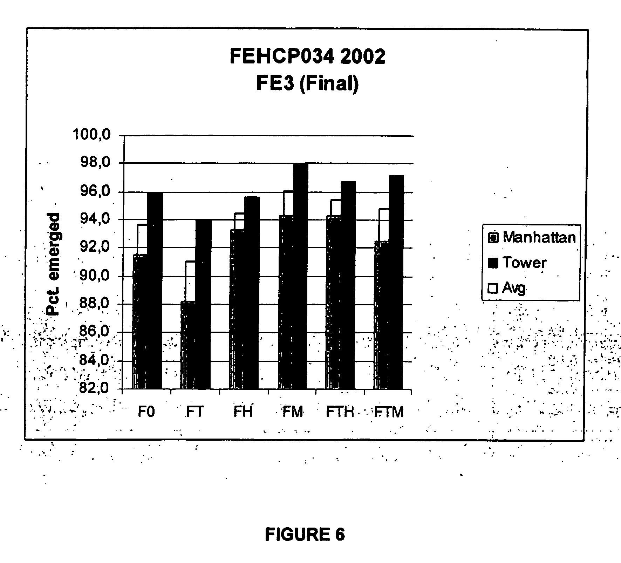

Figure 6 shows the final emergence of sugar beet seeds treated in accordance with Example 3.

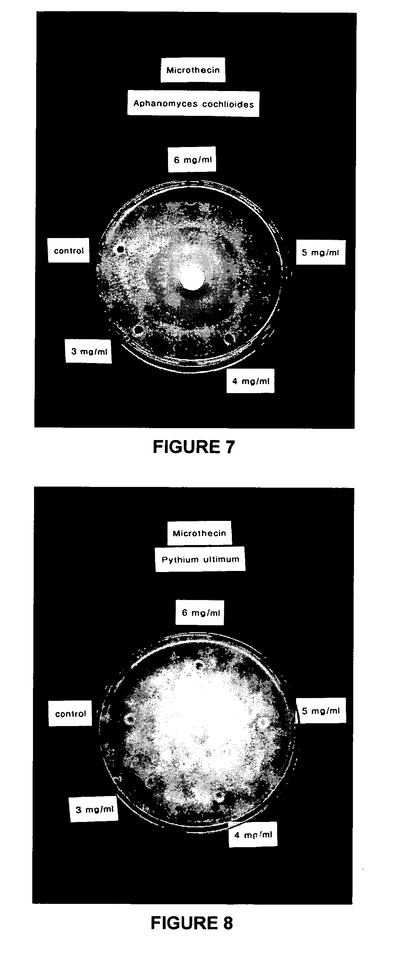

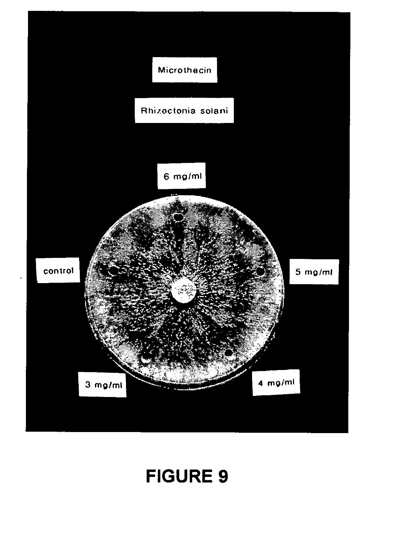

Figure 7 shows the screening effect of microthecin in different concentrations against the sugar beet root rot causing pathogen *Aphanomyces cochlioides.* Figures 8 and 9 show the screening effect of microthecin in different concentrations against the sugar beet root rot causing pathogens *Pythium ultimum* and *Rhizoctonia solani* respectively.

### EXAMPLES

### Pyranosome Dehydratase purified from the fungus Phanerochaete chrysosporium

*Phanerochaete chrysosporium* (white rot fungus) is a biotechnologically important fungus due to its higher growth optimum temperature (40°C) and its ability to produce a range of extracellular oxidative enzymes. Accordingly, this fungus has been used for treatment of various wastes, including explosive contaminated materials, pesticides, and toxic wastes. Furthermore, *Phanerochaete chrysosporium* is the first *basidiomycete* genome to be sequenced (University of California and Department of Energy, USA).

In the search for enzymes that metabolise anhydrofructose (AF), a purified a heat-stable pyranosone dehydratase (PD) was obtained from *P. chrysosporium.* Studies have shown that this purified PD not only uses AF as substrate, but uses it more efficiently than its natural substrate, glucosone. Furthermore, the product was shown to be microthecin, an antifungal useful in plant protection.

The N-terminal sequence of PD, and the endo-N-terminal sequences of PD after hydrolysis with two proteinases were elucidated. Together these account for 332 amino acids or 37% of the full length of the PD protein based on the assumption that it has a Mr of 97kDa.

Through database search using the above partial amino acid sequences on the fungal genome, the full length PD gene was identified in Scaffold 62 (Figure 4). The transcription start and stop codens together with 3 introns were identified (Figure 4). It appears that the purified PD is N-terminal 7-amino acid truncated, but still functional. Since the enzyme PD has not been found in culture medium, it may not have a signal peptide.

### Assay methods

### Measuring of the PD activity

The reaction mixture consisted of 25 µl of anhydrofructose solution (3.0%), 10 µl PD preparation, 93 µl 0.1 M sodium phosphate (pH 6.5), and water to a final volume of 1 ml. The reaction was mixed and scanned between 190 and 320 nm at room temperature (22 °C) every 5 min or after 30 min on a Perkin Elmer Lambda 18 uv/vis spectrophotometer. Absorbance values at 265 and 230 nm were recorded. One activity unit of PD is defined as the increase of 0.01 of absorbance unit at 230 nm at 22 °C per min.

A protein assay was carried out using the Bio-Rad Method (Bradford method) using the reagent and instructions form Bio-Rad laboratories [Peterson, GL: Determination of total protein, Methods Enzymol. 91, 95-119 (1983)]

TLC for separation of glucosone, AF and microthecin was performed as described before using a solvent system of ethylacetate, acetic acid, methanol and water (12:3:3:2) [Yu S, Ahmad T, Pedersén M, Kenne L: α-1,4-Glucan lyase, a new class of starch/glycogen degrading enzyme. III. Substrate specificity, mode of action, and cleavage mechanism, Biochim Biophys Acta 1244: 1-9 (1995)]. A Merck silica gel 60 (20x20cm) plate with a thickness of 0,15 mm was used. 1,5-Anhydro-D-fructose was assayed by the DNS method [Yu S, Olsen CE, Marcussen J: Methods for the assay of 1,5-anhydro-D-fructose and α-1,4-glucan lyase, Carbohydr. Res. 305: 73-82 (1998)].

### Purification of PD

The purification procedure used was essentially the same as that described by Gabriel et al., (1993) except the strains used were different. In addition, an extra ammonium sulfate fractionation step was included. The strain used in this application was *Phanerochaete chrysosporium* from American Type Culture Collection (ATCC 32629) and (ATCC 24725), while the strain used by Gabriel et al (1993) was *Phanerochaete chrysosporium* k-3 obtained from a Czechish collection centre.

The cell-free extract of *Phanerochaete crysosporium* was brought up to 55% ammonium sulphate saturation. It was then blended gently for 2 hours and centrifuged for 20 minutes at 4°C at 10000xg. The precipitate that had the PD activity was dissolved in the same volume of extraction buffer, centrifuged again and the supernatant was then used for the purification of PD using the procedure described by Gabriel et al. (1993).

The purification of PD procedure was followed by SDS-PAGE, and native-PAGE using PhastSystem (Pharmacia) using 8-25 % gradient gels according to the manufacturer's instructions. Visualization of protein bands on the gels was made with Coomassie brilliant blue staining (PhastGel Blue R). From Fig. 1A, PD1 is estimated to have a molecule mass 97 kDa it had a similar migration rate as the protein marker phosphorylase b (97.4 kDa).

### Amino acid sequencing

The purified PD was used for amino acid sequencing. Amino acid sequencing of PD was performed as described earlier [Yu. S.; Christensen TMIE, Kragh KM, Bojsen K, Marcussen J: Efficient purification, characterization and partial amino acid sequencing of two α-1,4-glucan lyases from fungi. Biochim Biophys Acta 1339: 311-320 (1997)]. PD was first partially hydrolyzed with proteinases. The generated peptide fragments were separated on HPLC. Each individual polypeptide was collected, molecule-mass determined by mass spectrometer, and sequenced on an Applied Biosystems 476A sequencer using pulsed-liquid fast cycles. PD was also further characterized for its pH and temperature optimum, ion requirements for activity, stability and other kinetic properties.

### Amino acid sequences obtained from Pyranosome Dehydratase purified from the fungus Phanerochaete chrysosporium.

The following amino acid sequences are obtained either by trypsin or endoproteinase LysC digestion. Peptide purification is achieved by reverse phase HPLC and molecular weight information is generated by MALDI-TOF mass spectrometry. The sequences obtained are then compared to the DNA sequences found in the White Rot Genome *(Phanerochaete chrysosporium)* project undertaken by The University of California. Sequence similarity alignment is done using the BLAST algorithm.

### All peptides producing significant alignments are found in Scaffold 62

### LysC peptides

### Peptide 27.3 (N terminal)

KPHCEPEQPAALPLFQPQLVQGGRPDXYWVEAFPFRSDSSK V possible heterogeneity

This peptide is found from base pair 38620 -38742. There is a start codon at base pair 38599 and at 38317 indicating a possible signal peptide. Independent confirmation that this is the N terminal of the protein is achieved by sequencing protein PD2, an isozyme.

The X at residue 27 is G in the data base, this fits well with the MS data.
MSc + = 4669.10 MSo+= 4668.01 -0.023%

### N-terminal

The N-terminal of Pyranosone dehydratase isozyme I (PDI) was found to be as follows:
KPHXEPEQPAALPLFQPQLVV(Q)GGRPDXY
X is unknown. V(Q) means it could be either V or Q or both (due to heterogeneity).

The N-terminal sequence above (Peptide 27.3) was isozyme II (PDII). The N-terminals of PDI and PDII are very similar or the same.

### Peptide 31.4 b

SDIQMFVNPYATTNNQSSXWTPVSLAKLDFPVAMHYADITK D possible heterogenity

This peptide is found from base pair 38788-38963. The data base sequence is interrupted by an intron from base pair 38836-38889. The sequence of residues 28-41 is confirmed by trypsin peptide 8.4

The X at residue 19 is S in the data base sequence, this fits with the MS data.
MSc+ = 4591.22 MSo+= 4591.55+0.007%

### Trypsin peptides

### Peptide 6a

### VSWLENPGELR

This peptide is found from base pair 39096-39128.
MSc+ =1300.44 MSo+ = 1300.45 + 0.001%

### Peptide 5

### DGVDCLWYDGAR

This peptide is found from base pair 39426-39461
MSc+ = 1427.48 MSo+ = 1427.48

### LysC peptides

### Peptide 27.4a

### PAGSPTGIVRAEWTRHVLDVFGXLXXK

This peptide is found from base pair 39673-39753
The three X's are PNG in the data base , this fits well with the MS data.
MSc+ = 2876.27 MSo+ = 2876.80 +0.021%

### Peptide 29.4.8

HTGSIHQVVCADIDGDGEDEFLVAMMGADPPDFQRTGVWCYK
This peptide is found from base pair 39754-39879
MSc+ = 4727.13 MSo+ = 4727.70 +0.012%

### Peptide 13.11

### TEMEFLDVAGK

This peptide is found from base pair 40244-40276
MSc+ = 1240.42 MSo+ = 1240.53 + 0.009%

### Peptide 14.2

### KLTLVVLPPFARLDVERNVSGVK

This peptide is found from base pair 40277-40345
MSc+= 2552.08 MSo+ = 25551.35 - 0.029%

### Trypsin peptide

### Peptide 10.5

### SMDELVAHNLFPAYVPDSVR

This peptide is found from base pair 40526-40585
MSc+ = 2259.55 MSo+ = 2259.77 + 0.009%

### LysC peptide

### Peptide 31.4a

### NDATDGTPVLALLDLDGGPSPQAWNISHVPPGTDMYEIAHAK

This peptide is found from base pair 41293-41469 and contains an intron from base pair 41362-41416
MSc+=4289.73 MSo+=4289.45 - 0.007%

### Peptide 2b

### TGSLVCARWPPVK

This peptide is found from base pair 41470-41508
MSc+ = 1471.71 MSo+ = 1472.62 +0.062%

### Peptide 2a

### NQRVAGTHSPAAMGLTSRWAVTK

This peptide is found from base pair 41509-41577
MSc+ = 2440.71 MSo+ = 2441.58 + 0.036%

### Peptide 11.3

### GQITFRLPEAPDHGPLFLSVSAIRHQ

This peptide is found from base pair 41641-41718
MSc+ = 2888.34 MSo+ = 2888.25 - 0.031%
This peptide does not end with K which is an indication of the C terminal. The sequence is also followed by a stop codon.

The molecular weight of this protein is approximately 97 KD. Based on the assumption that the average molecular weight of an amino acid is 110, the expected number of residues would be 880, which would give a total number of base pairs of 2640.

The number of base pairs calculated from the data base sequence is 3100. The two known introns comprise of 53 and 54 base pairs so if it is assumed that this figure is normal then the data base sequence is expected to contain about 8 introns.

The total number of residues sequenced here is 332 amino acids, which accounts for 37% of the protein.

### Example 1: Use of 1,5-anhydro-D-fructose and PD for the production of microthecin

The reaction mixture consisted of 1,5-Anhydro-D-fructose 5µl (3.0%), PD preparation 5µl, 65µl sodium phosphate buffer (pH 6.0) and water to a final volume of 0.7 ml. The reaction was monitored by scanning between 350-190 nm. Reaction time at zero min was used as blank. The absorbance peak at around 230 nm indicates the formation of microthecin. The absorbance at 265 nm indicate the first formation of an intermediate from AF before it converts to microthecin.

The microthecin formed was further confirmed by relative migration rate on TLC and its conversion of 2-furyhydoroxymethylketone that exhibits a typical absorbance peak at 275 nm [Baute M.-A. et al., 1986].

In larger scale production of microthecin, AF used was from 0.4% to 20%. The reaction was followed by AF disappearing from the reaction mixture using the DNS method [Yu. S.; Christensen TMIE, Kragh KM, Bojsen K, Marcussen J, Biochim Biophys Acta 1339: 311-320 (1997)]. The formation of microthecin was monitored at 265nm and its shift to 230nm, and was further monitored by TLC method.

1.5-Anhydro-D-fructose is found to be a much better substrate for the pyranosone dehydratase (PD) than for its natural substrate glucosone. The Vmax is around 4.7 times higher with AF than with glucosone (Table 1).

**Table 1**

| Final substrate concentration (µg/ml reaction mixture) | OD226nm using AF as substrate | OD226nm using Glucosone as substrate |
|---|---|---|
| 13.7 | 0.308 | 0.069 |
| 27.4 | 0.534 | 0.104 |
| 41.1 | 0.76 | 0.141 |
| 68.4 | 1.246 | 0.238 |
| 95.8 | 1.764 | 0.323 |
| 137 | 2.43 | 0.484 |
| 205 | 2.943 | 0.634 |

The reaction system consisted of AF or glucosone 1-15µl, 25µl sodium phosphate buffer (6.5. 0.1M), water, 1.4µl PD to a final volume of 200µl. The reaction was performed at 22°C for 5.5 hours. The formation of microthecin from AF and cortalcerone from glucosone were monitorered at 226nm.

### Example 2: Production of Cortalcerone

Cortalcerone may be produced in one step by incubating a starch-type substrate, such starch, waxy starch, dextrins, with starch hydrolases, such amyloglucosidase and a debranching enzyme or cyclodextrin transferase, pyranose 2-oxidase, and PD. After incubation Cortalcerone can be separated from the reaction mixture by ultrafiltation using membrane cut-off of 300-30,000, preferably 10,000.

### Example 3: Use of 1,5-anhydro-D-fructose, PD and ascopyrone P synthase for the production of APP

The reaction mixture consisted of 1,5-Anhydro-D-fructose 50µl (3.0%), PD preparation 5µl, ascopyrone P synthase 5 µl, 0.1 ml sodium phosphate buffer (pH 6.0) and water to a final volume of 0.8 ml. The reaction was monitored by the formation of APP at 289 nm spectrophotometrically. The reaction temperature was 22 °C and reaction time was 24 hours. At the end of 90% of AF had been converted to APP. The structure of APP was confirmed using NMR as described earlier [WO 00/56838 filed 16/3/00, claiming priority from GB9906457.8, filed 19/3/99].

### Expression of PD gene

The PD gene may be expressed in a production organism such as *Pichia pastoris, Aspergillus niger,* and *Hansenulla polymorph* by techniques well known in the art and referenced hereinbefore in the description.

### Antibody production

Antibodies were raised against the amino acid of the present invention by injecting rabbits with the purified enzyme and isolating the immunoglobulins from antiserum according to procedures described according to N Harboe and A Ingild ("Immunization, Isolation of Immunoglobulins, Estimation of Antibody Titre" In A Manual of Quantitative Immunoelectrophoresis, Methods and Applications, NH Axelsen, et al (eds.), Universitetsforlaget, Oslo, 1973) and by T G Cooper ("The Tools of Biochemistry", John Wiley & Sons, New York, 1977).

### Microthecin as an Anti-Fungal

Fungal growth in plant causes enormous economical damages. Examples are their damage to sugar beet seedlings and their leaves. As soon as the sugar beet seed is germinated in the soil it is immediately exposed to fungal attack by the species such as *Rhizoctonia solani, Pythium ultimum, Aphanomyces cochlioides.* In the present invention, it was found microthecin was able to inhibit the growth of these disease-causing fungi. Hence, the seeds of economical crops, sugar as sugar beet seeds are coated with a paste containing microthecin at 50-2000 ppm and dried before use for planting. Alternatively, aqueous solution of microthecin may be directly sprayed on the plant and its leaves.

### Experimental

Basic microthecin solution: 24 mg/ml Batch no. Mic20011016

### Dilutions used:

| Dilution factor | Concentration |
|---|---|
| 5 | 4.8 mg/ml |
| 10 | 2.4 mg/ml |
| 20 | 1.2 mg/ml |
| 50 | 0.48 mg/ml |
| 100 | 0.24 mg/ml |

All solutions were filtered through a 0.22 µm filter for sterilisation.

The solutions were tested against the following fungi:

| Fungus | Disease of sugar beet |
|---|---|
| *Rhizoctonia solani* | Root rot |
| *Pythium ultimum* | Root rot |
| *Aphanomyces cochlioides* | Root rot |
| *Cercospora beticola* | Leaf spot |

A circular plug (diameter 10 mm) of fresh mycelium was placed at the centre on a petri-dish (diameter 9 cm) containing PDA medium. (PDA= Potato dextrose agar Difco no. 213400). Wells with a diameter of 5 mm were cut along the periphery of the agar plate. In each well were placed 50 µl of a test solution. Alternatively, 20 µl of each test solution were placed directly on the agar along the periphery of the plate. Also, 50µl of each test solution were placed directly on top of the fungal mycelium plug.

The agar plates were placed at room temperature in daylight, but protected from direct sunlight.

The reaction (inhibition zones) of the fungi to the test substance was judged as follows:
- *Rhizoctonia solani*:: after 2-3 days of growth
- *Pythium ultimum*:: after 1-2 days of growth
- *Aphanomyces cochlioides*:: after 3-4 days of growth
- *Cercospora beticola*: after 3-4 weeks of growth

### Results

Microthecin as a fungal growth regulator was inhibitory against *Rhizoctonia solani, Pythium ultimum, Aphanomyces cochlioides* and *Cercospora beticola.* The minimum inhibition concentration (mic) of microthecin against these fungi were 240, 480, 1200 and 2400 ppm, respectively.

### Example 4: Effect of Microthecin on pelleted sugar beet seeds

The effect of microthecin on the plant pathogenic fungi *Pythium ultimum, Rhizoctonia solani* and *Aphanomyces cochlioides in vitro* was investigated by screening for growth inhibition of the pathogens on agar-plates (Figures 6, 7, 8).

Figure 7 shows the screening effect of microthecin in different concentrations against *Aphanomyces cochlioides,* whereas Figures 8 and 9 show the screening effect against *Pythium ultimum* and *Rhizoctonia solani* respectively. In each case, microthecin was dissolved in water and placed in wells in the periphery. An agar block containing the pathogen was placed in the centre. The pathogens were allowed to grow out on the PDA-agar plates for 3-5 days. These investigations showed that microthecin in very low concentrations was able to reduce the growth of *Aphanomyces.*

Similar tests with other microorganisms showed that Microthecin has no effect on *Cercospora.* Pseudomonads (*P. fluorescens* DS96.578, *P. mendocina* DS98.124) are slightly affected, whereas it has no effect on the growth of Bacillus (*B. Pumilus* DS96.734, *B. megaterium* DS98.124).

Based on these findings, the efficiency of microthecin was further investigated in a field emergence trial. The trial was sown relatively late giving it a higher chance for presence of the pathogen *Aphanomyces* in the trial field.

### Materials and methods

| | | | Pellet TKW | |
|---|---|---|---|---|
| 1. | Manhattan | CAC-7-2306 kb5, 3,0-4,25mm, | 19,2 (7) | 19,1 (1) |
| 2. | Tower | MIT-1-0290 kb5, 3,0-4,25mm. | 17,3 (8) | 17,8 (2) |

Seeds were pelleted with standard P1 pelleting mass with (1,2) or without (7,8)Thiram.

### Standard seed coating:

- Inner coating:: 0,3gai/U microthecin as a 0,5% solution in water or
14,7gai/U Hymexazol.
60gai/U Imidacloprid.
Standard metallic green seed cover film.

The following combinations were included in the trial:
- R F0: Without fungicides
- R FT: With Thiram (in pellet)
- R FH: With Hymexazol
- R FM: With Microthecin
- R P1 STD: With Thiram (in pellet) + Hymexazol
- R FTM: With Thiram (in pellet) + Microthecin

- Trial place: Bukkehave, DK. (4 reps, 200 seeds/plot)
- Trial sown: 21.05.2002
- 1. Count: 28.05.2002 (speed)
- 2. Count: 29.05.2002 (speed)
- 3. Count: 24.06.2002 (final)

### Results

Lab and field emergence figures can be found in Table 2 (Trial FEHCP034 *Aphanomyces).* The final emergence is shown in Figure 6.

**Table 2**

| | | | | | | | | | | | TSV | | | | | | Relative FE | | |
|---|---|---|---|---|---|---|---|---|---|---|---|---|---|---|---|---|---|---|---|
| Entry FE name | FEn o | Variety | Ty pe | 3d | 4d | 4d>1 5 | 7d | MM | Tvi | Abn | 7d | 14d | 21d | FE1 | FE2 | FE3 | 1 | 2 | 3 |
| Aphanom -yces PLACE: Buk | | | | | | | | | | | | | | | | | | | |
| 1 R F0 | 700 | 1 Manhattan | P | | 83 | 9 | 99 | 100 | 0,3 | 0,3 | | | | 36,3 | 96,5 | 183,0 | | | |
| 1 R F0 | 701 | 2 Tower | P | | 92 | 41 | 100 | 99 | 1 | 0 | | | | 33,3 | 98,7 | 191,7 | | | |
| 1 R F0 | | | | | 87,5 | 25,0 | 99,5 | 100 | 0,7 | 0,2 | | | | 34,8 | 97,6 | 187,3 | 100 | 100 | 99 |
| 2 R FT | 702 | 1 Manhattan | P | | 82 | 10 | 99 | 100 | 0,3 | 0 | | | | 38,0 | 100,8 | 176,3 | | | |
| 2 R FT | 703 | 2 Tower | P | | 94 | 40 | 99 | 99 | 0,5 | 0 | | | | 38,5 | 108,5 | 188,0 | | | |
| 2 R FT | | | | | 88,0 | 25,0 | 99,0 | 100 | 0,4 | 0,0 | | | | 38,3 | 104,6 | 182,1 | 110 | 108 | 97 |
| 3 R FH | 704 | 1 Manhattan | P | | 88 | 6 | 100 | 99 | 0,5 | 0 | | | | 23,8 | 77,8 | 186,5 | | | |
| 3 R FH | 705 | 2 Tower | P | | 87 | 24 | 99 | 99 | 0,8 | 0,3 | | | | 28,3 | 94,3 | 191,3 | | | |
| 3 R FH | | | | | 87,5 | 15,0 | 99,5 | 99 | 0,7 | 0,2 | | | | 26,0 | 86,0 | 188,9 | 75 | 88 | 100 |
| 4 R FM | 706 | 1 Manhattan | P | | 68 | 15 | 96 | 100 | 0 | 0,3 | | | | 37,0 | 101,3 | 188,5 | | | |
| 4 R FM | 707 | 2 Tower | P | | 76 | 37 | 97 | 99 | 0,5 | 0 | | | | 38,3 | 103,5 | 195,8 | | | |
| 4 R FM | | | | | 72,0 | 26,0 | 96,5 | 100 | 0,3 | 0,2 | | | | 37,6 | 102,4 | 192,1 | 108 | 105 | 102 |
| 5 STD P1 | 1 | 1 Manhattan | P | | 82 | 3 | 99 | 100 | 0 | 0 | 95 | 88 | 90 | 30,0 | 82,8 | 188,5 | | | |
| 5 STD P1 | 2 | 2 Tower | P | | 88 | 15 | 99 | 100 | 0 | 0 | 116 | 84 | 81 | 35,0 | 94,3 | 193,3 | | | |
| 5 STD P1 | | | | | 85,0 | 9,0 | 99,0 | 100 | 0,0 | 0,0 | 105, | 86,0 | 85,5 | 32,5 | 88,5 | 190,9 | 94 | 91 | 101 |
| 6 R FTM | 708 | 1 Manhattan | P | | 64 | 13 | 100 | 100 | 0 | 0 | | | | 39,3 | 98,5 | 185,0 | | | |
| 6 R FTM | 709 | 2 Tower | P | | 71 | 45 | 99 | 99 | 0,5 | 0 | | | | 39,5 | 110,8 | 194,3 | | | |
| 6 R FTM | | | | | 67,5 | 29,0 | 99,5 | 100 | 0,3 | 0,0 | | | | 39,4 | 104,6 | 189,6 | 113 | 108 | 101 |
| Opsummering for 'Place'=Buk (12 detaljeposter) Gnsnt | | | | 3d | 4d | 4d>1 | 7d | MM | Tvil | Abn | 7d | 14d | 21d | FE1 | FE2 | FE3 | | | |
| | | | | | 81,3 | 5 | 98,8 | 100 | 0,4 | 0,1 | 105, | 86,0 | 85,5 | 34,8 | 97,3 | 188,5 | | | |
| | | | | | | 21,5 | | | | | | | | | | | | | |

The results of the lab studies indicate that the inclusion of microthecin decreases the speed of laboratory germination (4d), but this is not reflected in the 4d>15mm figures. This is the opposite effect of Hymexazol that has a low 4d>15mm germination.

With regard to the speed of germination, the field emergence trials indicate that pellets containing Hymexazol - either alone, or in combination with Thiram - germinate relatively slow (as expected from the 4d>15mm lab germination). Pellets containing microthecin show a speed of germination comparable with pellets only containing Thiram.

In contrast to the fast germinating Thiram containing pellets the pellets containing Microthecin show a high final germination (comparable with Hymexazol containing pellets).

Although the actual attack by root rot causing pathogens was rather limited, the 4% (approximately) missing plantlets in the FT-plots arise from attack of plantlets by pathogens that can be controlled by Hymexazol (most probably *Aphanomyces*). The final number of plantlets in the FT-plots are lower than the number of plantlets in the F0 (no fungicides) plots. This can be explained by the action of Thiram, that controls other microbes, but not *Aphanomyces,* thereby allowing easier access of *Aphanomyces* to the plantlets.

The microthecin containing pellets are the only pellets that both show a fast germination and a high final germination. It is believed that microthecin might therefore be an alternative to the rather expensive chemical Hymexazol.

All publications mentioned in the above specification are herein incorporated by reference. Various modifications and variations of the described methods and systems of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in molecular biology or related fields are intended to be within the scope of the following claims.

Further aspect of the invention are set forth in the following numbered paragraphs:
1. An isolated polypeptide comprising at least one amino acid sequence selected from the following:
   (i) KPHCEPEQPAALPLFQPQLVQGGRPDXYWVEAFPFRSDSSK or KPHXEPEQPAALPLFQPQLVV(Q)GGRPDXY;
   (ii) SDIQMFVNPYATTNNQSSXWTPVSLAKLDFPVAMHYADITK;
   (iii) VSWLENPGELR;
   (iv) DGVDCLWYDGAR;
   (v) PAGSPTGIVRAEWTRHVLDVFGXLXXK ;
   (vi) HTGSIHQVVCADIDGDGEDEFLVAMMGADPPDFQRTGVWCYK;
   (vii) TEMEFLDVAGK;
   (viii) KLTLVVLPPFARLDVERNVSGVK;
   (ix) SMDELVAHNLFPAYVPDSVR;
   (x) NDATDGTPVLALLDLDGGPSPQAWNISHVPPGTDMYEIAHAK;
   (xi) TGSLVCARWPPVK;
   (xii) NQRVAGTHSPAAMGLTSRWAVTK;
   (xiii) GQITFRLPEAPDHGPLFLSVSAIRHQ;
   where X is an unknown amino acid residue; or a variant, homologue or derivative thereof.
2. A polypeptide according to paragraph 1 which comprises at least one sequence selected from paragraph 1.
3. A polypeptide according to paragraph 1 or paragraph 2 which has pyranosone dehydratase activity.
4. A polypeptide that is immunologically reactive with an antibody raised against a purified polypeptide according to any one of paragraphs 1 to 3.
5. An isolated polynucleotide encoding a polypeptide according to any one of paragraphs 1 to 4 or a variant, homologue, fragment or derivative thereof.
6. An isolated polynucleotide according to paragraph 5 which is selected from:
   (i) a polynucleotide comprising the nucleotide sequence of SEQ ID No. 1 or the complement thereof;
   (ii) a polynucleotide comprising a nucleotide sequence capable of hybridising to the nucleotide sequence of SEQ ID No. 1, or a fragment thereof;
   (iii) a polynucleotide comprising a nucleotide sequence capable of hybridising to the complement of the nucleotide sequence of SEQ ID. No. 1; and
   (iv) a polynucleotide comprising a polynucleotide sequence which is degenerate as a result of the genetic code to the polynucleotide of SEQ ID No. 1.
7. A nucleotide sequence according to any one of paragraphs 5, 6 or 7 wherein the nucleotide sequence is obtainable from *Phanerochaete chrysosporium, Polyporus obtusus* or *Corticium caeruleum.*
8. A nucleotide sequence according to any one of paragraphs 5, 6 or 7 wherein the nucleotide sequence is obtainable from the order of *Pezizales, Auriculariales, Aphyllophorales, Agaricales* or *Gracilariales.*
9. A nucleotide sequence according to any one of paragraphs 5, 6 or 7 wherein the nucleotide sequence is obtainable from the *Aleuria aurantia, Peziza badia, P. succosa, Sarcophaera eximia, Morchella conica, M costata, M elata, M esculenta, M esculenta var. rotunda, M hortensis, Gyromitra infula, Auricularia mesenterica, Pulcherricium caeruleum, Peniophora quercina, Phanerochaete sordida, Vuilleminia comedens, Stereum gausapatum, S. sanguinolentum, Lopharia spadicea, Sparassis laminosa, Boletopsis subsquamosa, Bjerkandera adusta, Trichaptum biformis, Cerrena unicolor, Pycnoporus cinnabarinus, P. sanguineus, Junghunia nitida. Ramaria flava, Clavulinopsis helvola, C. helvola var. geoglossoides, V. pulchra, Clitocybe cyathiformis, C. dicolor, C. gibba, C. odora, Lepista caespitosa, L inversa, L. luscina, L. nebularis, Mycena seynii, Pleurocybella porrigens, Marasmius oreales, Inocybe pyriodora, Gracilaria varrucosa, Gracilaria tenuistipitata, Gracilariopsis sp,* or *Gracilariopsis lemaneiformis.*
10. An isolated polynucleotide which is selected from:
   (i) a polynucleotide comprising the nucleotide sequence of SEQ ID No. 1 or the complement thereof;
   (ii) a polynucleotide comprising a nucleotide sequence capable of hybridising to the nucleotide sequence of SEQ ID No. 1, or a fragment thereof;
   (iii) a polynucleotide comprising a nucleotide sequence capable of hybridising to the complement of the nucleotide sequence of SEQ ID. No. 1; and
   (iv) a polynucleotide comprising a polynucleotide sequence which is degenerate as a result of the genetic code to the polynucleotide of SEQ ID No. 1.
11. A construct comprising the nucleotide sequence according to any one of paragraphs 5 to 10.
12. A vector comprising the nucleotide sequence according to any one of paragraphs 5 to 10.
13. An expression vector comprising a polynucleotide sequence according to any one of paragraphs 5 to 10 operably linked to a regulatory sequence capable of directing expression of said polynucleotide in a host cell.
14. A host cell into which has been incorporated the nucleotide sequence according to any one of paragraphs 5 to 10.
15. An isolated polypeptide encoded by the polynucleotide sequence of SEQ ID NO.1, or a variant, homologue, fragment or derivative thereof.
16. An isolated polypeptide according to paragraph 15 which has up to 7 amino acids removed from the N-terminus.
17. An isolated polypeptide according to paragraph 15 or paragraph 16 having at least 75% identity to a polypeptide sequence encoded by SEQ ID NO.1.
18. An antibody capable of binding a polypeptide according to any one of paragraphs 1 to 4 or 15 to 17.
19. A method of preparing polypeptide according to any one of paragraphs 1 to 4 or 15 to 17 wherein said process comprises expressing the nucleotide sequence according to any one of paragraphs 5 to 10, and optionally isolating and/or purifying same.
20. A process for preparing microthecin using a polypeptide according to any one of paragraphs 1 to 4 or 15 to 17.
21. A process for preparing ascopyrone P using a polypeptide according to any one of paragraphs 1 to 4 or 15 to 17.
22. A process according to paragraph 20 or paragraph 21 comprising reacting said polypeptide with 1,5-anhydro-D-fructose. ,
23. A process according to paragraph 21 wherein said process further comprises the use of APP synthase in the preparation of ascopyrone P.
24. A process according to paragraph 23 comprising reacting APP synthase and said polypeptide with 1,5-anhydro-D-fructose.
25. A process according to paragraph 20 which comprises contacting said polypeptide with glucan lyase and dextrins starch.
26. A process for preparing cortalcerone using a polypeptide according to any one of paragraphs 1 to 4 or 15 to 17.
27. A process according to paragraph 26 comprising reacting said polypeptide with glucosone.
28. A process according to paragraph 26 comprising reacting said polypeptide with glucose and pyranose 2-oxidase.
29. A process for preparing microthecin comprising reacting pyranosone dehydratase with 1,5-anhydro-D-fructose.
30. A process for preparing microthecin comprising reacting pyranosone dehydratase with glucose and dextrins starch.
31. A process for preparing ascopyrone P comprising reacting pyranosone dehydratase and APP synthase with 1,5-anhydro-D-fructose.
32. A process for preparing cortalcerone comprising reacting pyranosone dehydratase with glucosone.
33. A process for preparing cortalcerone comprising reacting pyranosone dehydratase with glucose and pyranose 2-oxidase.
34. Use of microthecin, cortalcerone, or derivatives or isomers thereof, in preventing and/or inhibiting the growth of, and/or killing the pathogen *Aphanomyces.*
35. Use according to paragraph 34 wherein the pathogen is *Aphanomyces cochlioides.*
36. Use according to paragraph 34 or paragraph 35 wherein the derivative of microthecin is 2-furyl-hydroxymethyl-ketone or 4-deoxy-*glycero*-hexo-2,3-diluose.
37. Use according to paragraph 34 or paragraph 35 wherein the derivative of cortalcerone is 2-furylglyoxal.
38. Use according to any one of paragraphs 34 to 37 in the treatment of plants or plant seeds.
39. Use of microthecin, cortalcerone, or derivatives or isomers thereof, as a plant or seed protectant.
37. Use according to any one of paragraphs 34 to 39 in the treatment of sugar beet seeds.
38. An enzyme having pyranosone dehydratase activity substantially as described herein and with reference to the accompanying Examples.
42. A nucleotide sequence encoding an enzyme having pyranosone dehydratase activity substantially as described herein and with reference to the accompanying Examples.
43. A process for preparing ascopyrone P substantially as described herein and with reference to the accompanying Examples.
44. A process for preparing microthecin substantially as described herein and with reference to the accompanying Examples.
45. A process for preparing cortalcerone substantially as described herein and with reference to the accompanying Examples.
46. A method of preventing and/or inhibiting the growth of, and/or killing the pathogen *Aphanomyces* substantially as described herein with reference to the accompanying Examples.

## Claims

1. Use of cortalcerone, microthecin, or derivatives or isomers thereof, in preventing and/or inhibiting the growth of, and/or killing the pathogen *Aphanomyces.*

2. Use according to claim 1 wherein the pathogen is *Aphanomyces cochlioides.*

3. Use according to claim 1 or claim 2 wherein the derivative of cortalcerone is 2-furylglyoxal.

4. Use according to claim 1 or claim 2 wherein the derivative of microthecin is 2-furyl-hydroxymethyl-ketone or 4-deoxy-*glycero*-hexo-2,3-diluose.

5. Use according to any one of claims 1 to 4 in the treatment of plants or plant seeds.

6. Use of cortalcerone, microthecin, or derivatives or isomers thereof, as a plant or seed protectant.

7. Use according to any one of claims 1 to 6 in the treatment of sugar beet seeds.

8. An isolated polypeptide comprising at least one amino acid sequence selected from the following:
(ii) KPHCEPEQPAALPLFQPQLVQGGRPDXYWVEAFPFRSDSSK or KPHXEPEQPAALPLFQPQLVV(Q)GGRPDXY;
(ii) SDIQMFVNPYATTNNQSSXWTPVSLAKLDFPVAMHYADITK;
(iii) VSWLENPGELR;
(iv) DGVDCLWYDGAR;
(v) PAGSPTGIVRAEWTRHVLDVFGXLXXK;
(vi) HTGSIHQVVCADIDGDGEDEFLVAMMGADPPDFQRTGVWCYK;
(vii) TEMEFLDVAGK;
(viii) KLTLVVLPPFARLDVERNVSGVK;
(ix) SMDELVAHNLFPAYVPDSVR;
(x) NDATDGTPVLALLDLDGGPSPQAWNISHVPPGTDMYEIAHAK;
(xi) TGSLVCARWPPVK;
(xii) NQRVAGTHSPAAMGLTSRWAVTK;
(xiv) GQITFRLPEAPDHGPLFLSVSAIRHQ;
where X is an unknown amino acid residue; or a variant, homologue or derivative thereof.

9. A polypeptide according to claim 8 which comprises at least one sequence selected from claim 8.

10. A polypeptide according to claim 8 or claim 9 which has pyranosone dehydratase activity.

11. A polypeptide that is immunologically reactive with an antibody raised against a purified polypeptide according to any one of claims 8 to 10.

12. An isolated polynucleotide encoding a polypeptide according to any one of claims 8 to 11 or a variant, homologue, fragment or derivative thereof.

13. An isolated polynucleotide according to claim 12 which is selected from:
(v) a polynucleotide comprising the nucleotide sequence of SEQ ID No. 1 or the complement thereof;
(vi) a polynucleotide comprising a nucleotide sequence capable of hybridising to the nucleotide sequence of SEQ ID No. 1, or a fragment thereof;
(vii) a polynucleotide comprising a nucleotide sequence capable of hybridising to the complement of the nucleotide sequence of SEQ ID. No. 1; and
(viii) a polynucleotide comprising a polynucleotide sequence which is degenerate as a result of the genetic code to the polynucleotide of SEQ ID No. 1.

14. A nucleotide sequence according to any one of claims 12 or 13 wherein the nucleotide sequence is obtainable from *Phanerochaete chrysosporium, Polyporus obtusus* or *Corticium caeruleum.*

15. A nucleotide sequence according to any one of claims 12 or 13 wherein the nucleotide sequence is obtainable from the order of *Pezizales, Auriculariales, Aphyllophorales, Agaricales* or *Gracilariales.*

16. A nucleotide sequence according to any one of claims 12 or 13 wherein the nucleotide sequence is obtainable from the *Aleuria aurantia, Peziza badia, P. succosa, Sarcophaera eximia, Morchella conica, M costata, M elata, M esculenta, M esculenta var. rotunda, M hortensis, Gyromitra infula, Auricularia mesenterica, Pulcherricium caeruleum, Peniophora quercina, Phanerochaete sordida, Vuilleminia comedens, Stereum gausapatum, S. sanguinolentum, Lopharia spadicea, Sparassis laminosa, Boletopsis subsquamosa, Bjerkandera adusta, Trichaptum biformis, Cerrena unicolor, Pycnoporus cinnabarinus, P. sanguineus, Junghunia nitida. Ramaria flava, Clavulinopsis helvola, C. helvola var. geoglossoides, V pulchra, Clitocybe cyathiformis, C. dicolor, C. gibba, C. odora, Lepista caespitosa, L inversa, L. luscina, L. nebularis, Mycena seynii, Pleurocybella porrigens, Marasmius oreales, Inocybe pyriodora, Gracilaria varrucosa, Gracilaria tenuistipitata, Gracilariopsis sp,* or *Gracilariopsis lemaneiformis.*

17. An isolated polynucleotide which is selected from:
(v) a polynucleotide comprising the nucleotide sequence of SEQ ID No. 1 or the complement thereof;
(vi) a polynucleotide comprising a nucleotide sequence capable of hybridising to the nucleotide sequence of SEQ ID No. 1, or a fragment thereof;
(vii) a polynucleotide comprising a nucleotide sequence capable of hybridising to the complement of the nucleotide sequence of SEQ ID. No. 1; and
(viii) a polynucleotide comprising a polynucleotide sequence which is degenerate as a result of the genetic code to the polynucleotide of SEQ ID No. 1.

18. A construct comprising the nucleotide sequence according to any one of claims 12 to 17.

19. A vector comprising the nucleotide sequence according to any one of claims 12 to 17.

20. An expression vector comprising a polynucleotide sequence according to any one of claims 12 to 17 operably linked to a regulatory sequence capable of directing expression of said polynucleotide in a host cell.

21. A host cell into which has been incorporated the nucleotide sequence according to any one of claims 12 to 17.

22. An isolated polypeptide encoded by the polynucleotide sequence of SEQ ID NO.1, or a variant, homologue, fragment or derivative thereof.

23. An isolated polypeptide according to claim 22 which has up to 7 amino acids removed from the N-terminus.

24. An isolated polypeptide according to claim 22 or claim 23 having at least 75% identity to a polypeptide sequence encoded by SEQ ID NO.1.

25. An antibody capable of binding a polypeptide according to any one of claims 8 to 11 or 22 to 24.

26. A method of preparing polypeptide according to any one of claims 8 to 11 or 22 to 24 wherein said process comprises expressing the nucleotide sequence according to any one of claims 12 to 17, and optionally isolating and/or purifying same.

27. A process for preparing ascopyrone P using a polypeptide according to any one of claims 8 to 11 or 22 to 24.

28. A process according to claim 27 comprising reacting said polypeptide with 1,5-anhydro-D-fructose.

29. A process according to claim 27 wherein said process further comprises the use of APP synthase in the preparation of ascopyrone P.

30. A process according to claim 29 comprising reacting APP synthase and said polypeptide with 1,5-anhydro-D-fructose.

31. A process for preparing cortalcerone using a polypeptide according to any one of claims 8 to 11 or 22 to 24.

32. A process according to claim 31 comprising reacting said polypeptide with glucosone.

33. A process according to claim 31 comprising reacting said polypeptide with glucose and pyranose 2-oxidase.

34. A process for preparing microthecin comprising reacting pyranosone dehydratase with 1,5-anhydro-D-fructose.

35. A process for preparing microthecin comprising reacting pyranosone dehydratase with glucose and dextrins starch.

36. A process for preparing ascopyrone P comprising reacting pyranosone dehydratase and APP synthase with 1,5-anhydro-D-fructose.

37. A process for preparing cortalcerone comprising reacting pyranosone dehydratase with glucosone.

38. A process for preparing cortalcerone comprising reacting pyranosone dehydratase with glucose and pyranose 2-oxidase.
